Europäisches Patentamt

**European Patent Office**

**Office européen des brevets**

(11) Publication number: **0 232 707**
**A1**

(12) **EUROPEAN PATENT APPLICATION**

(21) Application number: 87100107.9

(22) Date of filing: 07.01.87

(51) Int. Cl.⁴: **C 12 N 15/00,** C 07 H 21/04,
C 12 N 5/00, C 12 P 21/00,
C 07 K 13/00, C 07 K 15/00
// (C12N15/00,C12R1:85),
(C12N15/00,C12R1:185)

(30) Priority: 09.01.86 JP 2633/86
18.12.86 JP 302698/86

(43) Date of publication of application: 19.08.87
Bulletin 87/34

(84) Designated Contracting States: BE CH DE FR GB IT LI SE

(71) Applicant: AJINOMOTO CO., INC., 5-8,
Kyobashi 1-chome, Chuo-ku, Tokyo 104 (JP)
Applicant: Taniguchi, Tadatsugu, No. 19-A-207,
Mihogaoka, Ibaragi-shi Osaka-fu (JP)

(72) Inventor: Tadatsugu, Taniguchi, 19-A-207, Mihogaoka,
Ibaragi-shi Osaka-fu (JP)
Inventor: Gen Yamada, No. 1-5-3, Gakuen Minami,
Nara-shi, Nara-ken (JP)
Inventor: Junji, Hamuro Centr.Res.Lab. Ajinomoto Co.
Inc., No. 1-1, Suzuki-cho Kawasaki-ku Kawasaki-shi,
Kanagawa-ken (JP)
Inventor: Shinsuke, Taki Centr.Res.Lab. Ajinomoto
Co.Inc., No.1-1, Suzuki-cho Kawasaki-ku Kawasaki-shi,
Kanagawa-ken (JP)
Inventor: Hiroshi, Matsui Centr.Res.Lab. Ajinomoto
Co.Inc., No. 1-1, Suzuki-cho Kawasaki-ku, Kawasaki-shi,
Kanagawa-ken (JP)
Inventor: Nobukazu, Kashima Centr.Res.Lab. Ajinomoto
Co.Inc., No. 1-1, Suzuki-cho Kawasaki-ku, Kawasaki-shi,
Kanagawa-ken (JP)

(74) Representative: Strehl, Schübel-Hopf, Groening, Schulz,
Widenmayerstrasse 17 Postfach 22 03 45,
D-8000 München 22 (DE)

(54) Human hematopoietic cell growth potentiating factor.

(57) A gene coding for a polypeptide having human hematopoietic cell growth potentiating factor activity is provided. Said
gene contains a portion in which sites cleaved with restriction
endonuclease are arranged in the order of Sph I, Pst I and Ban I
from the 5'-end of the coding sequence. According to the
present invention recombinant DNA harboring the genetic
DNA sequence, living cell lines carrying said DNA, and a
method for the production of human hematopoietic cell growth
potentiating factor (HCGPF) using the living cell lines and a
polypeptide having HCGPF activity are provided.

EP 0 232 707 A1

*1*

HUMAN HEMATOPOIETIC CELL GROWTH POTENTIATING FACTOR

Detailed Description of the Invention

(Field of Industrial Application)

The present invention relates to a gene coding for polypeptide of a factor with growth potentiating activity for hematopoietic cells, particularly bone marrow cells (hereafter HCGPF: Hematopoietic Cell Growth Potentiating Factor), recombinant DNA harboring the gene, living cell lines having the DNA , a method for production of HCGPF using the living cell lines and polypeptide having HCGPF activity.

This HCGPF potentiates the growth of bone marrow cells, thymocytes, fetal liver cells even by single use thereof but when used in combination with other hematopoietic cell growth factor HCGF, the growth of these hematopoietic cells is markedly accelerated but the presence of such a factor is unknown hitherto.

The present inventors have already identified a gene for a factor which induces the growth of mouse IL3-dependent cell line FDC-P cells (Japanese Patent Application No. 2633/86). The present inventors have now succeeded in producing this factor as a single cytokine using the gene and in purifying the factor up to a single protein and could identify it as a substance for the first time. As described above, it has been confirmed that this factor acts widely on hematopoietic cells such as bone marrow cells, thymocytes,

0232707

- 2 -

fetal    liver cells, etc. and when used in combination with other factors termed HCGFs, this factor induces more remarkable growth of hematopoietic cells and the present invention has thus been come to accomplish.

This HCGPF exhibits immune control and hematopoietic control functions over a wide range based on the aforesaid activity and its utility has been strongly expected in the fields of immunodeficient diseases, autoimmune diseases, infectious diseases, hepatitis, nephritis, cancers, bone marrow transplantation, etc. At the same time, the factor is effective in potentiating effects when used in combination with other immunoactive substances, immunotherapeutic agents, lymphokines, cytokines, interferon, cell growth factors, chemotherapeutic agents, antibiotics or anti-viral agents as well as alleviating side effects of other drugs.

This factor is a novel substance having the utility as described above. This factor is not only expected to have utility in the medical field but this factor, the antibody to the factor and the receptor for the factor are also expected to be useful as diagnostic agents.

Further by the use of this substance, hematopoietic cells can be efficiently proliferated artificially using cell culture technique and the provision of the gene of this factor, a method for production thereof and of this factor as a substance results in great effects. It is particularly important to create a technique capable of providing this substance which is present only in a trace amount in the living human body, in a simple manner in large quantities using microorganisms, etc.

(Prior Art)

As factors collectively called a colony stimulating factor which directly acts on the growth of hematopoietic cells 3 kinds have recently been reported and their genes have been subjected to cloning (Gouzh, N.M. Et al., Nature, 309, 763 (1984); Kawasaki, E.S., et al., Science, 230, 291 (1985) and Nagata, S., et al., Nature, 319, 415 (1986)). These factors are simply referred to as GM-CSF, M-CSF and G-CSF, respectively.

In addition, multi-CSF as a colony stimulating factor which acts widely on hematopoietic cells is known with mice and the factor has already been subjected to genetic cloning (Fung, M.C., et al., Nature, 307, 233 (1984), Yokota, T., et al., Proc. Nat. Acad. Sci., USA, 81, 1070 (1984)).

Further with mouse, there has been found a lymphokine having such a specific action that acts on undifferentiated mouse T cells to induce 20α-hydroxysteroid dehydrogenase (hereafter referred to as "20αSDH") and differentiate into mature T cells, which is named interleukin 3 (hereafter referred to as "IL-3"). Further the biological activity of mouse IL-3 is believed to participate not only in differentiation and growth of cells such as neutrophilic granulocytes, megakaryocytes, mast cells, basophilic granulocytes, etc. but also in metabolism of sexual hormones and, its wide biological activity is greatly interesting (Immunological Rev., 63, 532 (1982)). For these reasons mouse IL-3 is also called multi-CSF.

Because of such a biological activity, this substance is expected to be useful over a wide range of diseases caused by immunological deficiency or abnormality and useful for clinical diagnosis of immune diseases and at the same time, useful as reagents for general investigation of biological and medical science.

However, IL-3 is merely clarified on its gene and protein structure with mice but with respect to human IL-3, it is even unclear if human IL-3 is present because there is no appropriate assay system for the activity and suitable IL-3-producing cells are unknown.

There is well known an activity that these human G-CSF, M-CSF, GM-CSF, mouse IL-3 (multi-CSF) act on stem cells in bone marrow at the stage of differentiation and maturation to produce colonies of hematopoietic cells and, their usefulness is being examined in areas of bone marrow transplantation and therapy of immunodeficient diseases.

Upon administration of such a factor to the living body, it is most desirable to apply to the living body in a biological concentration inherent thereto. External application to human body in a large dose merely results in occurrence of side effects and often accompanying difficulty in application as medicine, which is obviously noted from recent clinical cases of human interleukin 2 (IL-2).

A novel technique that is to be accomplished by the present invention is to identify and provide a substance having an activity of synergestically potentiating the growth of hematopoietic cells by the use in combination with these factors having colony forming action of these hematopoietic cells and by itself showing the action singly.

Accordingly, this factor has also human IL-3-like activity at the same time.

(Problem to be solved by the invention)

Essential means to make the HCGPF useful are to identify as a substance a factor for potentiating the growth of human hematopoietic cells (hereafter referred to as HCGPF) either in combination with such factors (interleukin 3 = multi-CSF, etc.) particpating in growth of hematopoietic cells, introduce a gene coding for this factor in cells of microorganisms, etc. and produce the factor.

Therefore, objects of the present invention are to identify the gene for this HCGPF, provide a method for production by introducing the gene in living cell line, produce HCGPF using living cell line containing recombinant DNA and identify this HCGPF as a novel substance.

(Means for solving the problem)

The present inventors have prepared human peripheral blood derived mononuclear cells in a conventional manner; their lymphocytes are stimulated for 48 hours in the presence of PHA and TPA, and RNAs are extracted by extraction of the thus activated lymphocytes with phenol in a conventinal manner. After precipitation with ethanol, mRNAs are collected by affinity chromatography using oligo dT-cellulose. In order to improve the purity of the mRNAs and fractionate the mRNAs by size, oligo dT-cellulose chromatography is repeated.

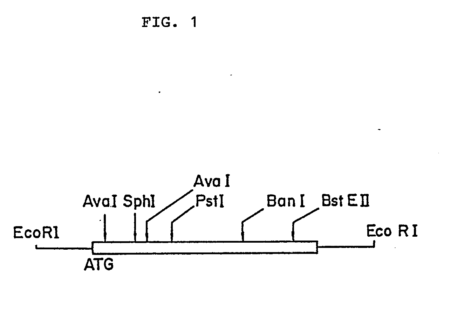

On the other hand, cDNA expression vector pDE-2 in monkey cells (COS cells) has been constructed (Japanese Patent Application No. 208645 filed October 4, 1984). Namely this pDE-2 is constructed using pML-R11G (Nature, 293, 79 (1981)) and pKCR (Proc. Natl. Acad. Sci., U.S.A., 78, 1527 (1981)) as shown in Figure 1 in which cDNA can be inserted in bidirectional SV40 early promoter and pDE-2 can be selected as being capable of replicating in E. coli and by ampicillin resistance.

This pDE-2 subjected to $^{C}$-tailing is mixed with the aforesaid double stranded cDNA subjected to G-tailing to perform aneating. The anealed DNA is introduced into competent E. coli MC1061 (J. Mol. Biol., 138, 179-207 (1980)), which is applied to agar plate of L medium to appear colonies.

The thus obtained colonies are divided into 192 groups of 32 colonies each followed by mix culture. Plasmid DNAs mixed with 32 kinds are purified in a conventional manner.

This plasmid mixture (32 kinds) is introduced into monkey cells (COS-7) (Cell, 23, 175-182 (1981)) and treated with chloroquine. After washing the cells, culture is performed for 48 hours. With respect to the culture supernatant, FDC-P cell growth activity is measured and a DNA mixture group having the activity is identified. DNA plasmid in the group containing the DNA mixture in which the activity has been recognized is purified as a single plasmid DNA of 32 and again introduced in COS cells. By assaying for the activity of the culture supernatant in a similar manner, clone p4-15 showing FDC-P cell growth activity, namely, IL-3-like activity, is identified. Then, cDNA contained in this clone is analyzed according to the dideoxy chain termination method (Sanger et al., Proc. Natl. Acad. Sci., U.S.A., 74, 5463-5467 (1977)) and the Maxam-Gilbert method (Meth. Enzym., 65, 510-580 (1980)), whereby the whole base sequence has been determined. It is clarified that the cDNA comprises base pairs of approximately 900 bp and codes for secretory protein having signal peptide. This cDNA and the structure of polypeptide presumed therefrom have not been found to date and are, therefore, quite novel. It is also assumed that the cDNA would be a novel lymphokine because messenger RNA corresponding to this cDNA expresses specifically on human lymphocytes activated by TPA and PHA.

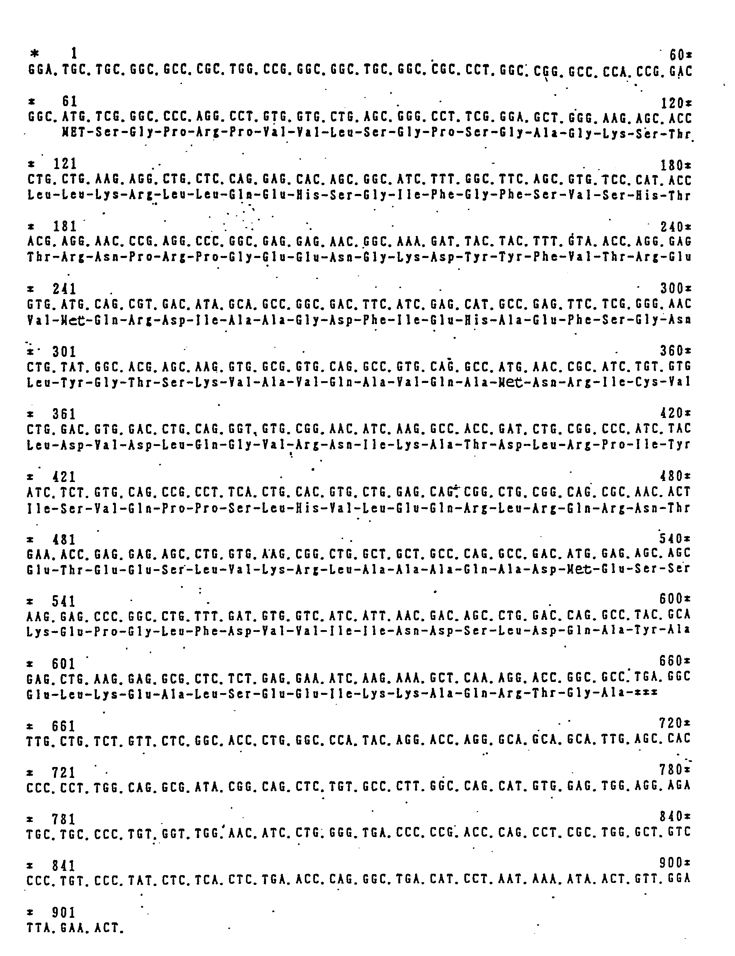

A cleavage map of cDNA insert with restriction enzyme endonuclease is shown in Figure 1. As shown in Figure 1, this cDNA has a structure cleaved with restriction enzyme endonuclease of Ava I, Pst I or Ban I. The base sequence of the cDNA is shown in Figure 2.

The DNA sequence of the cDNA insert holds a large open reading frame therein. The first ATG sequence (Kozak, M. Cell., 15, 1109-1123 (1978)) which is often a reading initiation sequence of eukaryote is present in nucleotide at the 64- to 66-position from the 5'-end thereof and 196 codons from the 655- to 657-position in the nucleotide site in which reading termination codon TGA is present are connected with this ATG. Linkage of A corresponding to the 3'-poly(A) end of mRNA is found at the cDNA end and nucleotide AATAAA (approximately 883 to 888) comprising 6 which is generally found in most of eucaryote mRNA is located frontward (Proudfoot, N.J. & Brownlee C.G., Nature, 263, 211-214 (1976)).

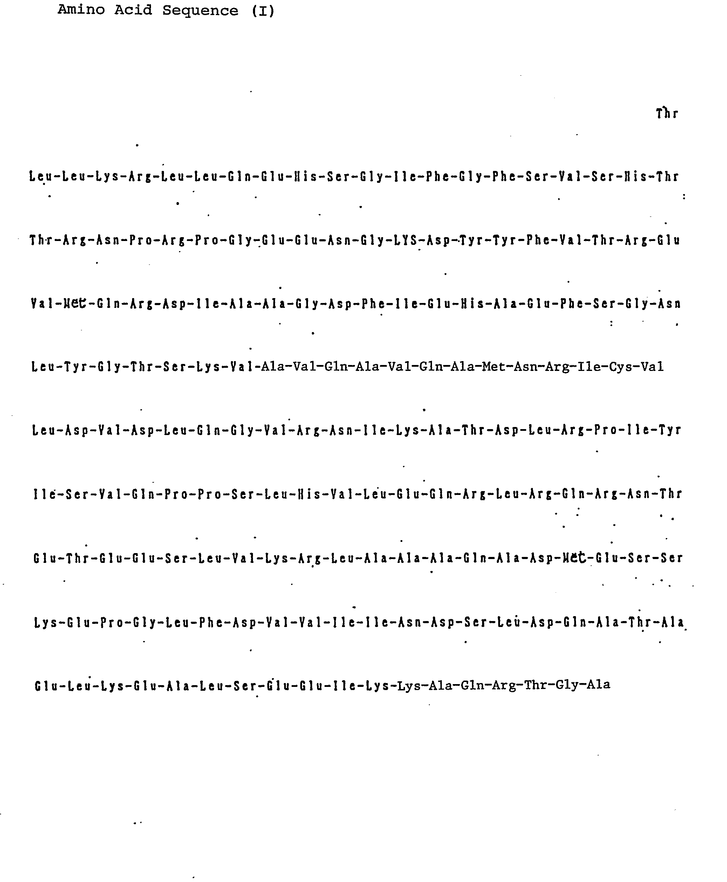

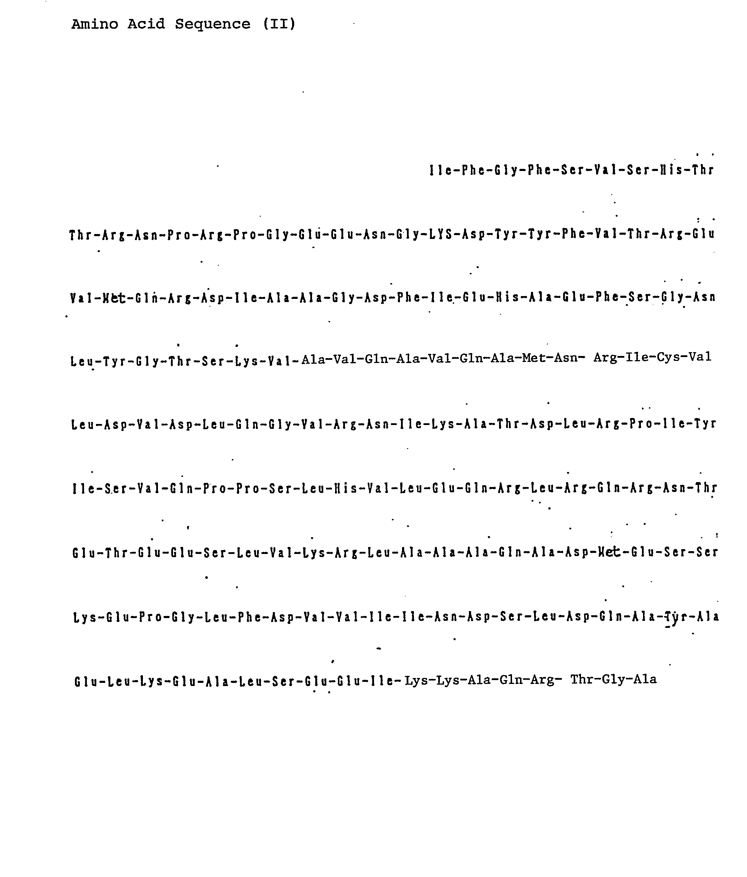

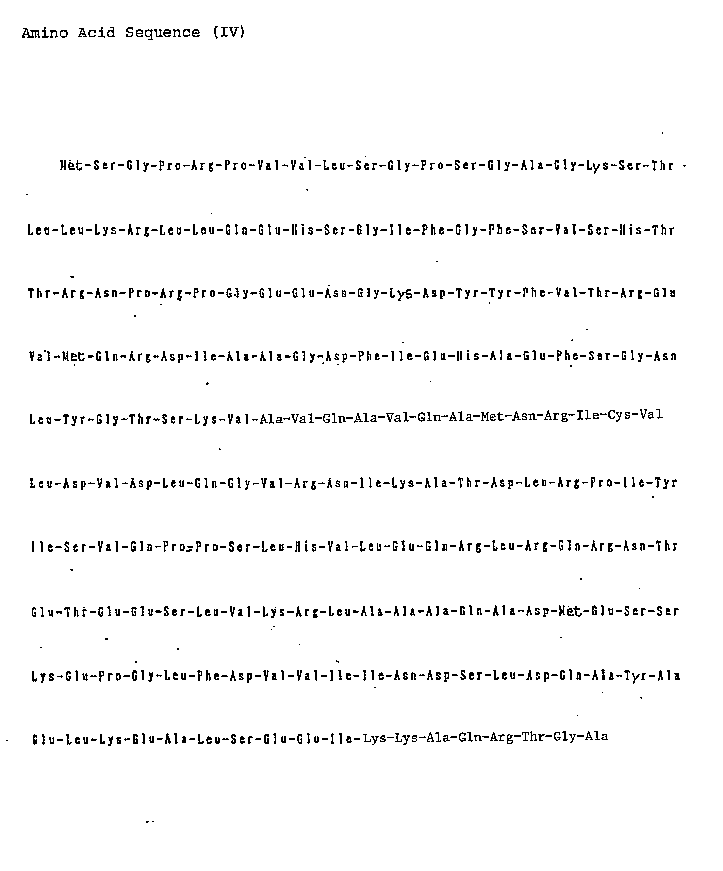

The amino acid sequence which cDNA codes for can be deduced as shown in Figure 5 (amino acid sequence III). In addition, polypeptide in amino acid sequence III is composed of 197 amino acids and as is reported to be found in most of secretory protein known to date (Blobel G., et al., Sym. Soc. Exp. Med., 33. 9-36 (1979)), the N-terminal area of the aforesaid deduced HCGPF polypeptide is hydrophobic as well. This region would take a role of signal peptide cleaved upon secretion of mature HCGPF. The cleavage occurs either between Ser and Thr at the 18- to 19-positions or between Gly and Ile at the 30- to 31-positions, whereby polypeptides having amino acid sequence I (Figure 3) and amino acid sequence II (Figure 4) are formed, respectively. This is because such cleavage sites are often found in other secretory proteins hitherto known (Blobel, G., et al., Symp. Soc. Exp. Med., 33, 9-36 (1979)). Therefore, mature HCGPF polypeptide is composed of 179 to 167 amino acids. Further as shown in Example 4, it has been confirmed that DNA fraction starting with ACC codon at the 118- to 120-positions in the base sequence, namely, code (amino acid sequence I in Figure 3) for polypeptide starting with Thr located at the

0232707

- 10 -

19-position expresses polypeptide having HCGPF activity. It has been confirmed that the DNA fraction starting from ATC at the 154- to 156-positions in the base sequence, namely, as shown in amino acid sequence II in Figure 4, the DNA fraction coding for polypeptide starting with Ile located at the 31-position expresses polypeptide having HCGPF activity as shown in Example 5.

It is known that genes of eukaryote show polytypic phenomenon as is known with human interferon genes (Taniguchi et al., Gene, 10, 11-15 (1980), Ono & Taniguchi, Proc. Natl. Acad. Sci., U.S.A., 77, 5305-5309 (1981); Gray et al., Nature, 295, 501-508 (1981)). By this polytypic phenomenon, some of amino acids of protein product are sometimes replaced; alternatively, any change does not occur even though the base sequence changes.

As is evident from the foregoing description, the gene of the present invention includes DNAs having the base sequence shown in Figure 2, DNA having a continuous base sequence from ATG sequence at the 64- to 66-positions to at least GCC sequence at the 652- to 654-positions, DNA having a continuous base sequence from ACC sequence at the 118- to 120-positions to GCC sequence, and DNA having a continous base sequence from ATC sequence at the 154- to 156-positions to at least GCC sequence. The gene of the present invention also includes DNA ending with GCC at the 652- to 654-positions and starting with G at the 1-position, DNA starting with ATG at the 64- to 66-positions and DNA starting with ATC sequence at the 118- to 120-positions. Further the gene of the present invention includes DNA ending with T at the 909-position and starting with A at the 1-position, DNA starting with ATG at the 64- to 66-positions, DNA starting with ACC at the 118- to 120-positions and DNA starting with ATC sequence at the 154- to 156-positions. The gene of the present invention also includes DNA ending with poly(A) and starting with ATG codon at the 64- to 66-positions, DNA starting with ACC sequence at the 118- to 120-positions and DNA starting with ATC sequence at the 154- to 156-positions. The present invention also includes the genes having base sequences corresponding to amino acid sequence I, amino acid sequence II and amino acid sequence III. In amino acid sequence III, polypeptide deficient in one or more amino acids or polypeptide in which one or more amino acids in amino acid sequence III is/are substituted with one or more amino acids may have HCGPF activity and therefore, genes coding for such polypeptides can be used in the present invention. Likewise even though one or more bases capable of expressing one or more amino acids to amino acid sequence I, amino acid sequence II or amino acid sequence III are additionally attached to the genes, such genes are included in the present invention unless the additional amino acids inhibit the expression of HCGPF activity. Even though it is a modification region having a supplemental amino acid sequence that inhibits the polypeptide function as HCGPF, it can be utilized as the gene of the present invention if a freshly supplemented region were to be removed. The same also applies to the case wherein DNA coding for supplementation of amino acids at the C-terminal of amino acid sequence I, amino acid sequence II or amino acid sequence III of the gene corresponding to amino acid sequence I, amino acid sequence II or amino acid sequence III is supplementarily bound to the 3'-end thereof. Therefore, the utilization of the gene coding for such polypeptide is included in the present invention.

Recombinant DNA producing HCGPF in living cells can be prepared by various methods below. For example, a sequence coding for HCGPF-cDNA is inserted downstream promoter sequence of expression vector. Alternatively, a cDNA fragment having a promoter sequence can be inserted upstream a sequence coding for HCGPF prior to or after the insertion of cDNA of expression vector.

Formation of eukaryote or prokaryote which expresses HCGPF-cDNA and produces HCGPF-polypeptide is described in detail below.

.In order to expsress HCGPF-cDNA in Escherichia coli, cDNA is bound to various bacteria promoters and thereafter, hybrid plasmid containing cDNA is formed upstream the promoters. The plasmid is transfected to, e.g., Escherichia coli HB101, whereby bacteria biosynthesizing protein having human HCGPF activity are cloned. Any bacterial promoter expresses HCGPF-cDNA as far as it is appropriately connected with cDNA. Expression of such cDNA will be later shown in the examples.

HCGPF-cDNA can be expressed even in yeast if it is incorporated in a suitable expression vector and the vector is introduced in host cells. Various shuttle vectors for expressing external gene in yeast have already been reported (Heitzman et al., Nature, 293, 717-722 (1981); Miyashita et al., Proc. Natl. Acad. Sci. U.S.A., 80, 1-5 (1983)). These vectors can be grown in both Escherichia coli and yeast. These vectors also include therein the promoter sequence of yeast gene. Fundamentally, expression vectors can all be utilized for expression of HCGPF-cDNA. When yeast is used, it is possible to enhance the level of HCGPF productivity, as compared to utilization of animal cells or bacteria. It is also possible to produce HCGPF containing sugar regions, which is effective for improving solubility of HCGPF.

Yeast-_Escherichia_ _coli_ shuttle vectors pAT77 and pAM82 are reported by Miyashita et al. (Proc. Natl. Acad. Sci. U.S.A., 80, 1-5 (1983)). Vector pAM82 is a derivative of pAT77. Both have an ars 1 marker (Stinchcomb, D.T., et al., Nature, 282, 39-43 (1979), 2 umori (Braoch, J.R., et al., Gene., 8, 121-133 (1979), leu2 (Ratzkin B. et al., Proc. Natl. Acad. Sci. U.S.A., 74, 474-491 (1989)) and have promoter go yeast acid phosphatase (APase). Both vectors have a DNA fraction composed of 3700 bases of pBR 322 having ampicillin resistant marker (APr) and an amplification origin. Apase promoter is induced by shifting phosphoric acid of a high concentration to a low concentration in a culture solution.

In order to insert the amplified DNA into other eucaryocyte, a vector suited for host cells may be bound to cDNA insert cleaved and separated from procaryocyte. Then eucaryocyte is transfected to synthesized vector followed by incubation.

The cells having inserted therein recombinant DNA are cultured to amplify the recombinant DNA or produce HCGPF polypeptide. This culture is performed in a conventional manner. For example, transfected yeast is aerobically cultured at 27 to 37°C at pH of 4 to 7 in medium containing carbon sources, nitrogen sources, inorganic salts and if necessary, organic nutrient sources such as vitamins, amino acids, etc. Transformed prokaryote such as Escherichia coli or Bacillus subtilis can also be cultured in a conventional manner.

The thus obtained HCGPF obtained herein is assumed to be human-derived lymphokine corresponding to mouse IL-3; however, it cannot be concluded that HCGPF would be human derived one corresponding to mouse IL-3 because the presence of human IL-3 is unclear yet and any authorized assay system of human IL-3 activity is unknown. In fact, the structure of this cDNA has no similarity to that of mouse IL-3 cDNA and, it is unlikely that both would be branched from the same ancestral gene.

Further FDC-P cells are cell lines conventionally used as IL-3-dependent cell lines in both domestic and foreign countries. It is also confirmed that FDC-P cells do not grow in the presence of other lymphokines. These activities are not found in; for example, interleukin 2, interleukin 1 and interferon. The proteins having amino acid sequences which the genetic sequence obtained herein codes for are heretofore unknown. The proteins are also different structurally from human G-CSF, M-CSF and GM-CSF which show activity somewhat similar to IL-3 and are substances different from so called selective colony stimulating factor (CSF). Therefore, the activity shown by the polypeptide that is expressed by the gene (cDNA) and assumed to be novel lymphokine obviously takes at least a part of the aforesaid human IL-3 activity.

The thus obtained human IL-3-like protein can be readily purified, singly or in combination, by salting-out, gel filtration, affinity chromatography, ion exchange chromatography, isoelectric electrophoresis, high speed liquid chromatography and other known methods for separation and purification of polypeptide.

The IL-3-like protein is expressed in COS cells in large quantities and its activity is assayed; the IL-3-like protein shows even singly growth inducing activity to mouse bone marrow cells, human bone marrow cells, thymocytes or mouse fetal liver cells. Apart from degree of the activity, it has been confirmed that the IL-3-like protein shows multi-CSF-like activity.

Further in the system using mouse bone marrow cells as target cells, the expression protein induces synergetically remarkable growth in the presence of mouse IL-3.

From the foregoing, this novel lymphokine polypeptide is to designate HCGPF as a factor that has multi-CSF-like activity likewise human IL-3 and acts synergetically with IL-3 to grow hematopoietic cells. The thus obtained HCGPF shows the same biochemical and biological

0232707

- 17 -

behavior as that of HCGPF obtained from mammalian cells and exhibit HCGPF activity. The HCGPF activity is stable even after treating with DNase or RNase or after thermally treating at 56°C for 30 minutes. The activity is stable at pH of 4 to 9.

(Effects)

HCGPF of the present invention has immune control function and hematopoietic control function and is thus applicable to the fields of immunodeficient diseases, infectious diseases, cancers, hepatitis, nephritis, bone marrow transplantation, etc. Further HCGPF of the present invention is thought to be effective for potentiationg effects when used in combination with other immunoactive substances, immunotherapeutic agents, lymphokine, cytokine, interferon, cell growth factors, chemotherapeutic agents, antibiotics or anti-viral agents and alleviating side effects of other medicines.

Hereafter the present invention will be described in detail with reference to the examples below.

- 18 -

Example 1

.(1) Human peripheral blood-derived cells were prepared in accordance with the method of Falkoff et.al.(J. Immunol. Method., 50, 39 (1982)) and the method of Taniguchi et.al.(Proc. Natl. Acad. Sci. U.S.A., 78, 3469-3472 (1981)). After stimmulating with 5 μg/ml of PHA and 5 ng/ml of TPA for 48 hours, the peripheral blood-derived cells ($3.4 \times 10^{10}$) were suspended in approximately 40 ml of RSB (10 mM Tris-HCl, pH 7.5, 10 mM NaCl, 1.5 mM $MgCl_2$). The cells were washed twice by centrifugal operation and resuspended in 200 ml of a PBS solution containing 10 mM of ribonucleoside-vanadyl complex.

Surfactant NP-40 was further added to the suspension in a final concentration of 0.1%. The mixture was gently agitated and the cell nuclei were removed by centrifugation at 3,000 rpm for 5 minutes at 4°C. After adding SDS (0.5%) and EDTA (5 mM) to the supernatant, an equal amount of water-saturated phenol was added to the mixture to perform extraction. After repeating the extraction with phenol 3 times, RNAs were precipitated with a 2-fold amount of cold ethanol. The precipitates were collected by centrifugation and dissolved in 10 mM Tris-HCl solution having pH of 7.5. The obtained RNAs were 40 mg.

Fractionation of mRNAs was carried out by affinity chromatography using oligo dT-cellulose (P.L. Biochemicals, Type 7). An adsorbing solution was a solution containing 20 mM Tris-HCl, 0.5 M NaCl, 1 mM EDTA and 0.5% SDS having pH of 7.5. After thoroughly washing a column with buffer (20 mM Tris-HCl, pH 7.5, 0.5 mM NaCl and 1 mM EDTA), elution was carried out using $H_2O$ and 10 nM Tris-HCl having pH of 7.5, alternatively. The mRNA obtained by the elution was 0.9 mg based on 33 mg of RNAs applied.

In order to further enhance the purity of the mRNA , affinity chromatography using oligo dT-cellulose was again repeated in a similar manner to give 0.45 mg of authentic mRNA .

(2) Next, cDNA library was made from the authentic mRNAs as follows and used for cDNA cloning.

The cDNA was synthesized fundamentally according to the Land et al method (Nucleic Acids Res., 9, 2551 (1981)).

(a) 50 mM of Tris-HCl (pH 8.3), 10 mM of $MgCl_2$, 0.1 M of KCl, 10 mM of dithiothreitol (DTT), 0.5 mM each of dATP, dGTP, dCTP and dTTP (including $^{32}$P radiation-labeled dCTP), 0.375 OD units of oligo $dT_{10}$, 50 µg of mRNA and 50 units of AMV reverse transcriptase (manufactured by Life Science Co., Ltd.) were mixed and the mixture was reacted at 41°C for 90 minutes. After completion of the reaction, the reaction mixture was treated with phenol and mRNA-cDNA hybrid was recovered as precipitates with ethanol. The precipitates were dissolved in 0.3N NaOH solution. The solution was settled at room temperature for 15 hours. Then an equal amount of 2 M Tris-HCl having pH of 7.5 was added thereto to neutralize. Thereafter the mixture was passed through a Sephadex G-50 column to recover cDNA. The recovered single stranded cDNA was approximately 10 µg.

(b)  0.1 M of potassium cacodylate (pH 7.2), 10 mM of DTT, 2 mM of $CaCl_2$, 0.5 mM of $^{32}P$-dCTP (specific activity, 1 x $10^6$ cpm/nmole), 10 µg of cDNA and 60 units of deoxynucleotidyl terminal transferase (BRL) were mixed. After incubating at 24°C for 23 minutes, the mixture was treated with phenol and passed through a Sephadex G-50 column to collect cDNA fractions and, 9.5 µg of dC-tailed cDNA was obtained as precipitates from ethanol. In the cDNA approximately 20 dCMP residues were added to the 3'-end thereof.

(c)  50 mM of potassium phosphate buffer (pH 7.5), 10 mM of $MgCl_2$, 10 mM of DTT, 1 mM each of dATP, dGTP, dCTP and dTTP (including $^3H$-labeled dCTP), 7.2 µg of oligo $dG_{10}$, 9.5 µg of single stranded cDNA and 50 units of DNA polymerase I (Klenow) (manufactured by BRL) were mixed and the mixture was reacted at 15°C for 6 hours.

After completion of the reaction, the reaction mixture

0232707

- 21 -

was treated with phenol and passed through a Sephadex G-50 column to recover double stranded cDNA fractions. As precipitates from ethanol, approximately 16 µg of DNA was recovered.

(d) Next, 33 mM of Tris-acetate (pH 7.9), 10 mM of magnesium acetate, 10 mM of DTT, 66 mM of potassium acetate, 0.1 mM each of dATP, dGTP and dTTP (excluding dCTP), 16 µg of double stranded cDNA and 22.5 units of T4 DNA polymerase (Takara) were mixed and the mixture was reacted at 37°C for 15 minutes to remove dC-tails attached to the 3'-end thereof.

After completion of the reaction, the reaction mixture was treated with phenol and passed through a Sephadex G-50 column to collect DNA fractions. As precipitates from ethanol, approximately 15.5 µg of DNA was recovered.

(e) Next, 50 mM of sodium acetate (pH 4.5), 0.2 M NaCl, 1mM of $ZnCl_2$ and 15.5 µg of double stranded cDNA was subjected to preliminary incubation at 37°C for 20 minutes and then 3 units of nuclease $S_1$ (manufactured by Sankyo Co., Ltd.) were added thereto followed by incubation at 37°C for further 15 minutes. After completion of the reaction, treatment with phenol was carried out twice and the system was passed through a Sephadex G-50 column to give 15 µg of double stranded cDNA.

(f) The obtained double stranded cDNA, 15 µg, were

fractionated by the sucrose density gradient centrifugal method (in a solution containing 50 mM of Tris-HCl and 1 mM of EDTA showing pH of 7.5 with 5 to 25% of sucrose density gradient, at 40,000 rpm and 4°C for 13 hours.) A part of the fractions was analyzed by autoradiogram according to agarose gel electrophoresis. Fractions of double stranded cDNA having a size of 600 bp or more were collected and double stranded cDNA was recovered by precipitation from ethanol. The recovered single stranded cDNA was approximately 7 μg.

(g) 0.1 M of potassium cacodylate (Tris base but pH was adjusted to 7.2), 10 mM of DTT, 2 mM of $CoCl_2$, 0.5 mM of $^{32}P$-dGTP (specific activity, 1 x $10^6$ cpm/nmole), 7 μg of double stranded cDNA and 50 units of deoxynucleotidyl terminal transferase (manufactured by BRL) were mixed. After incubating the mixture at 24°C for 20 minutes, the mixture was treated with phenol and passed through a Sephadex G-50 column to collect cDNA fractions. As precipitates from ethanol 6 μg of dG-tailed cDNA was obtained. In the cDNA, approximately 13 dGMP residues were added to the 3'-ends thereof.

(h) Next, 6 μg of dG-tailed cDNA was subjected to 1% agarose gel electrophoresis to fractionate by size. Namely, cDNA fractions having approximately 700 bp of more were adsorbed to DEAE-cellulose paper and then eluted with 20 mM

Tris-HCl solution of pH 7.5 containing 1.5 M of NaCl to recover the cDNA fractions. Recovery was effected by further treatment with phenol, treatment with chloroform and precipitation with ethanol to give approximately 1.9 µg of dG-tailed cDNA.

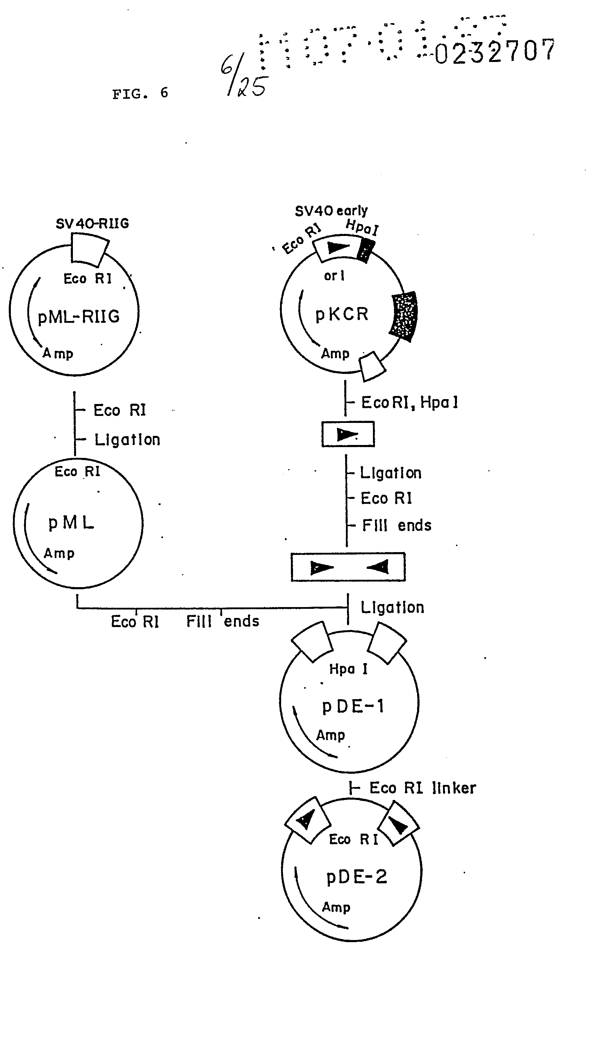

(3) On the other hand, as shown in Figure 6, cDNA expression vector pDE-2 in monkey cells (COS cells) was constructed. pDE-2 can be inserted into bidirectional SV 40 early promoter, is replicable and can be selected as ampicillin-resistance.

This pDE-2 was cleaved with EcoRI and cohesive ends were filled up with DNA polymerase I (Klenow). Then approximately 13 dC-tails were added to the aforesaid ds-cDNA at the 3'-ends thereof in quite the same manner as in the dG-tails added to the ds-cDNA at the 3'-ends thereof.

Next, 100 ng of the dC-tailed pDE-2 and 20 ng of dG-tailed ds-cDNA were mixed in a solution of pH 7.5 containing 0.1 M of NaCl and 1 mM of EDTA followed by incubation firstly at 65°C for 2 minutes, then at 45°C for 60 minutes, 37°C for 60 minutes and then at room temperature for 60 minutes. The thus annealed DNA was introduced into competent E. coli MC1061. Next, preparation of MC1061 competent cells and introduction thereof are shown below.

E. coli MC 1061 was inoculated on 100 ml of ψ medium

0232707

- 24 -

(2% tryptone, 0.5% yeast extract, 0.5% $MgSO_4 \cdot 7H_2O$, pH 7.6), which was shake cultured at 37°C until absorbance of the culture solution became approxametely 0.3 to 0.5 at 550 nm. After completion of the culture, the culture solution was kept at 0°C for 5 minutes and cells were collected by centrifugation. The cells were suspended in 40 ml of Tfb I (30 mM of potassium acetate, 100 mM of $CaCl_2$, 50 mM of $MnCl_2$, 15% glycerine, pH 5.8) and the suspension wa settled at 0°C for 5 minutes.

Again cells were collected by centrifugation. The cells were suspended in 4 ml of Tfb II (10 mM of MOPS or PIPES, 75 mM of $CaCl_2$, 10 mM of RbCl, 10% glycerine, pH 6.5) and the suspension was settled at 0°C for 15 minutes. The suspension was separately charged and kept at -70°C.

Next, 100 µl of the thus prepared competent cells were kept at 0°C for 15 minutes and 10 µl of the aforesaid authentic product obtained by annealing dG-tailed pDE-2 vector and dC-tailed cDNA and 90 µl of a solution containing 50 mM of $MgCl_2$ and 10 mM of $CaCl_2$ were mixed therewith. The mixture was settled at 0°C for 20 minutes. After heat treating at 37°C for 60 seconds, the mixture was kept at 0°C for 1 to 2 minutes and 7 ml of ψ medium was added thereto followed by shake culture at 37°C for 60 minutes. The culture solution was smeared on agar plate of L medium (1% of tryptone, 0.5% of yeast extract, 0.5% of NaCl) containing 25 µg/ml of ampicillin and 25 µg/ml of streptomycin. By incubation at 37°C overnight, colonies appeared.

(4) The appeared colonies were divided into 192 groups by 32 colonies each, which were inoculated on 100 ml of ψ medium containing 25 µg/ml of ampicillin and 25 µg/ml of streptomycin, respectively, followed by shake culture at 37°C for 5 to 7 hours. Next, 100 ml of fresh ψ medium containing chloramphenicol in a final concentration of 170 µg/ml was added to the culture solution followed by shake culture overnight.

The thus amplified plasmid DNA was purified as follows.

The culture solution was centrifuged to collect cells only. The cells were suspended in 5 ml of 50 mM Tris-HCl showing pH of 7.5. After freezing at -80°C, the system was fused and lysozyme (final concentration, 2 mg/ml) was added thereto and settled at 0°C for 10 minutes. EDTA (final concentration, 0.1 M) was further added thereto and settled at 0°C for 10 minutes. Thereafter Triton X-100 (final concentration, 0.1%) added thereto and settled at 0°C for 60 minutes. Then the ultracentrifugal separation was performed at 30,000 rpm for 30 minutes and the supernatant was treated with an equal amount of water-saturated phenol. The aqueous phase was further treated with an equal amount of chloroform and extracted. Rnase was added to the extract in a final concentration of 20 µg/ml followed by incubation at 37°C for 60 minutes.

Thereafter 0.2 volume of 5 M NaCl and 1/3 volume of polyethylene glycol were added to the system. After settling at 0°C for 60 minutes, DNA precipitates were recovered by centrifugation at 10,000 rpm for 20 minutes.

Next the precipitates were dissolved in 3.8 ml of water and 4 g of $CaCl_2$ was added to the solution to dissolve it. Then 200 µl of 10 mg/ml of EtBr was added followed by ultracentrifugal fractionation at 40,000 rpm for 16 hours at 20°C.

After completion of the centrifugation, extraction was performed 4 times with 1 to 2 volume of water-saturated n-butanol to remove EtBr. Then dialysis was performed in $H_2O$ to remove CsCl. Thereafter 1/10 volume of 3 M sodium acetate showing pH of 5.6 was added and 2 volume of cold ethanol was further added thereto followed by settling at -20°C overnight. The precipitates with ethanol were collected by centrifugation and then washed with 80% ethanol-aqueous solution. After thoroughly drying them, the precipitates were dissolved in 50 µl of 10 mM Tris-HCl having pH of 7.5. The solution was made a sample for monkey cell transfection.

**Example 2**

(1) Transfection of plasmid to monkey COS-7 cells

COS-7 cells were suspended in 1 x $10^5$/ml in 10% fetal bovine serum (FBS)-containing DMEM and 8 ml of the suspension was cultured at 37°C overnight in a Petri's dish in a 5% carbon dioxide gas incubator. Further 5 ml of fresh 10% FBS- containing DMEM was added and culture was continued at 37°C for 4 hours in the 5% carbon dioxide gas incubator. After completion of the culture, the supernatant was removed and the system was washed once with 5 ml of TBS (25 mM of Tris-HCl, pH 7.5, 130 mM of NaCl, 5 mM of KCl and 0.6 mM of $Na_2PO_4$).

A plasmid mixture (2.5 ml of TBS (+) (TBS added with 0.7 mM of $CaCl_2$ and 0.5 mM of $MgCl_2$)), 5 µg of plasmid DNA and 128 µl of 10 mg/ml DEAE-dextran) was added to the system and the mixture was incubated at 37°C for 1 hour in the 5% carbon dioxide gas incubator. After the supernatant was removed, the system was washed with 5 ml of TBS and 5 ml of 150 µM chloroquine-containing 10% FBS DMEM. After incubating at 37°C for 3 hours in the 5% carbon dioxide gas incubator, the supernatant was removed and the system was washed twice with 5 ml of TBS. Then 8 ml of 10% FBS -containing DMEM was added thereto followed by culturing at 37°C overnight in the 5% carbon dioxide gas incubator.

After the supernatant was removed, 8 ml of the same DMEM was added followed by incubation at 37°C for 2 days in the 5% carbon dioxide gas incubator. After the culture supernatant was centrifuged, the supernatant was made a sample for measurement of IL-3-like activity.

(2) Assay method and assay results

The proliferation activity of FDC-P cells was measured in accordance with the following method. In each cell of a 96-well tissue culture plate was charged 100 μl each of the culture supernatant of the COS cells, FDC-P cell proliferation activity of which was to be assayed and, 2-fold dilution was repeated in RPMI 1640 plus 10% FBS medium.

Then 100 μl each of the FDC-P cells which were IL-3-dependent cell lines were charged in each cell in a cell density of 1 x 10$^4$/100 μl. After incubation at 37°C for 18 hours FDC-P in 5% $CO_2$, 1 μCi of tritium thymidine was added to the culture solution. After performing pulsing for 6 hours, cells were harvested on glass fiber filter paper in a manner well known in the art. After a scintillator was added thereto, ß-radiation incorporated into the cells was counted. As a sample shows a high FDC-P cell proliferation activity, an amount of tritium thymidine incorporated into the cells, by which the amount of the FDC-P cell proliferation factor in the culture supernatant of the COS cells could be readily assayed quantitatively.

·The proliferation activity was assayed with respect to the 192 culture supernatants obtained by transfection of the aforesaid plasmid mixture to the COS cells according to this method and the results shown in Table 1 were obtained, wherein a significant activity was noted with the group designated No. 3-11.

## Table 1

| Sample | Amount of Tritium Thymidine Incorporated (cpm) | | | |
|---|---|---|---|---|
| | Dilution Degree of Sample | | | |
| | x2 | x4 | x8 | x16 |
| 3-11 | 2204 | 2282 | 2610 | 1106 |
| 1-1~1-32 | | | | |
| 2-1~2-32 | | | | |
| 3-1~3-10 | | | | |
| 3-12~3-32 | 296 | 258 | 252 | 220 |
| | ~532 | ~568 | ~572 | ~558 |
| 4-1~4-32 | | | | |
| 5-1~5-32 | | | | |
| 6-1~6-32 | | | | |
| Mouse IL3* | 8610 | 5308 | 2878 | 1492 |
| Gene-non-introduced COS supernatant (negative control) | 441 | 452 | 366 | 375 |
| Control (medium alone) | 360 | 466 | 452 | 534 |

* A 60-fold dilution of the culture supernatant of mouse IL-3-producing cells, WEH1-3, was used as a sample.

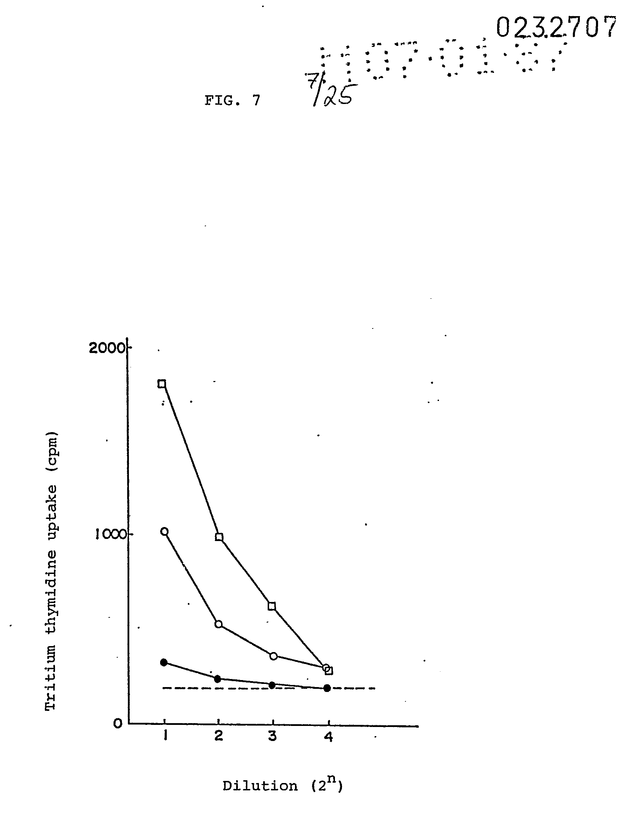

(3)  Group 3-11 was a mixture of 32 clones.  Thus the 32 clones were separately cultured and plasmid was prepared in quite the same manner as used in Example 1 (4) and transfected to COS cells in the same manner as used in the preceding (1).  With the culture supernatant, FDC-P cell growth activity was determined, whereby the growth activity was noted in one clone (15th clone, designated p4-15) (Figure 7).  It was identified that this clone had cDNA showing IL-3-like activity.

(4)  cDNA was excised from plasmid p4-15 using EcoR I and its base sequence was determined according to the dideoxy chain termination method using M13 and the Maxam-Gilbert method; it was presumed from its open reading frame that this HCGPF cDNA was composed of base pairs of approximately 900 bp and coded for precursor HCGPF protein composed of about 197 amino acids or 83 amino acids.

The base sequence (Figure 2) and presumed amino acid sequence (Figures 8 and 9) of this cDNA are shown.

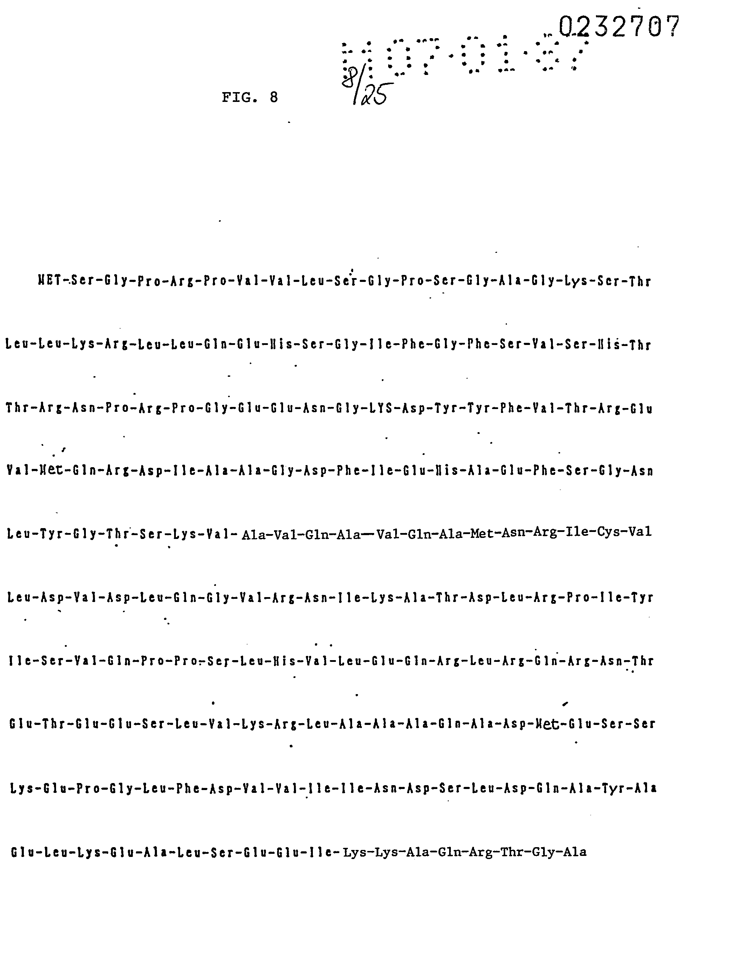

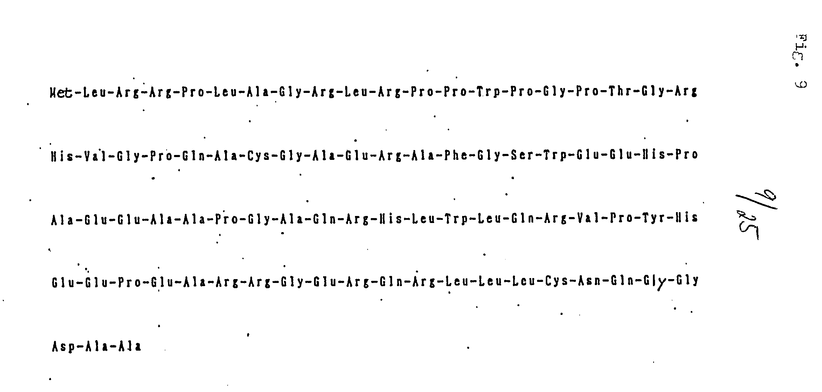

Namely, this cDNA is composed of 909 bases and the amino acid sequences shown in Figures 8 and 9 are assumed as those that the cDNA codes for.

Example 3

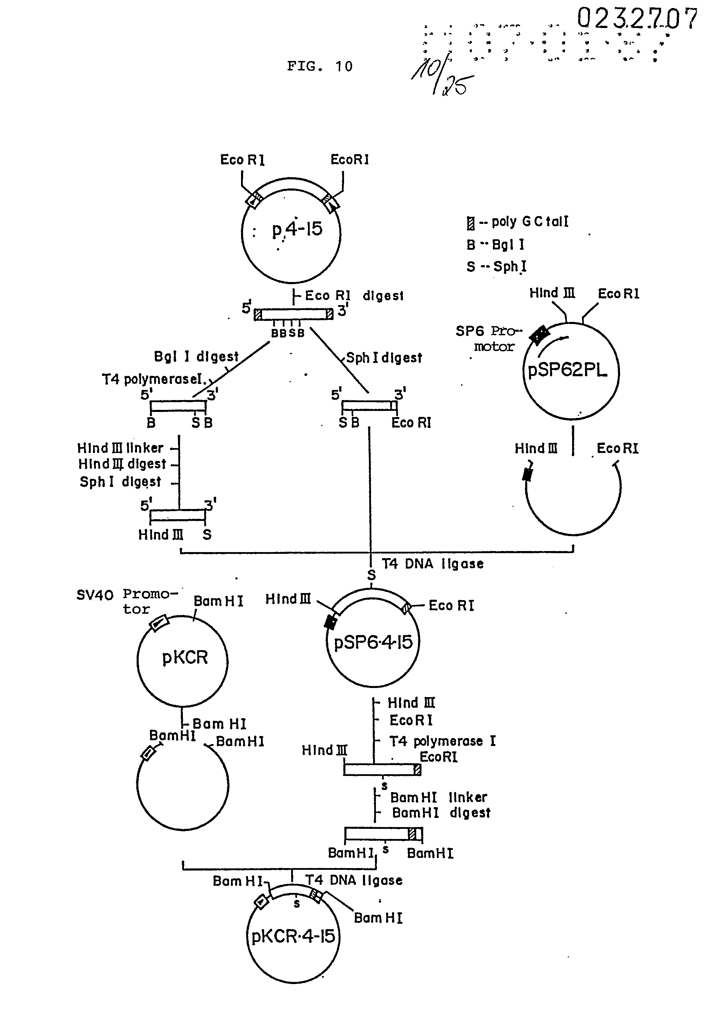

(1)  In order to express human HCGPF gene more efficiently, plasmid from which poly-GC tail contained in cDNA at the 5'-position thereof was excised was constructed as follows (Figure 10).

From plasmid p4-15 constructed in Example 1, cDNA was excised with restriction enzyme EcoR I and a part of them was cleaved with restriction enzyme Bgl I. The thus obtained Bgl I-Bgl I fragment of 396 base pairs was made smooth at the both ends thereof using T4 polymerase and then mixed with Hind III linker in a molar ratio of 1:1 followed by ligation using T4 DNA ligase. After the ligated fragment was recovered, they were cleaved with restriction enzymes Hind III and Sph I to give Hind III-Sph I fragment.

On the other hand, the remaining cDNA was cleaved with Sph I to give Sph I-EcoR I fragment. Further plasmid pSP62-PL (NEN) was cleaved with restriction enzymes Hind III and EcoR I to give DNA fragment containing SP6 promoter.

The thus obtained three fragments were mixed in a molar ratio of 1:1:1 and ligated using T4 DNA ligase. The thus obtained recombinant DNA was introduced into Escherichia coli HB 101 strains and a strain having ampicillin resistance was selected. From the separated strain plasmid was prepared. By cleavage with restriction enzyme and determination of base sequence around the ligated site, bacterial harboring pSP6 4-15 were selected.

Next, plasmid obtained from the bacteria was cleaved

0232707

- 32 -

with restriction enzymes Hind III and EcoR I to recover cDNA insert. After the cDNA was made smooth at the both ends thereof using T4 polymerase, it was mixed with BamH I linker in a molar ratio of 1:1 and the mixture was ligated using T4 ligase. The ligated fragment was mixed with pKCR (Glutzman, Y., Cell, 23, 175-182 (1981)) cleaved with restriction enzyme BamH I and the mixture was cleaved using T4 ligase. The thus obtained recombinant DNA was introduced into _Escherichia coli_ HB 101 strains and a strain having ampicillin resistance was selected. From the separated strain, plasmid was prepared. By cleavage with restriction enzyme, bacteria harboring plasmid pKCR 4-15 in which cDNA had been inserted in the order of EcoR I-BamH I-SpH I-Bgl I-BamH I were selected (pKCR 4-15). The pKCR 4-15/HB 101 was cultured in a conventional manner and plasmid pKCR 4-15 was obtained in a manner similar to the procedure in Example 1, (4).

Example 4

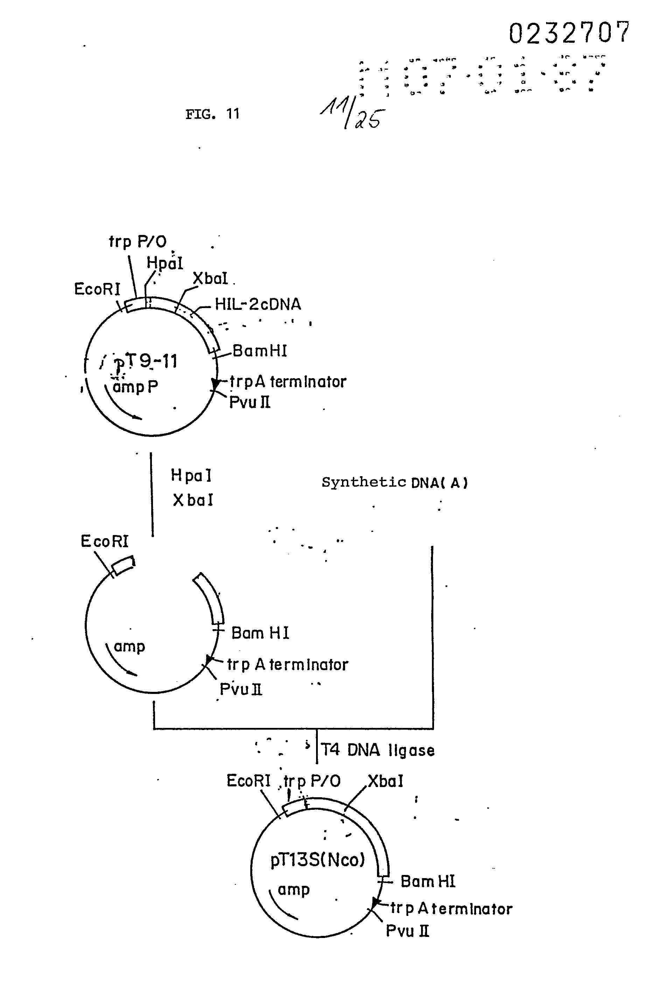

(1) Vector DNA used to express human HCGPF gene on prokaryote was constructed as follows (Figure 11).

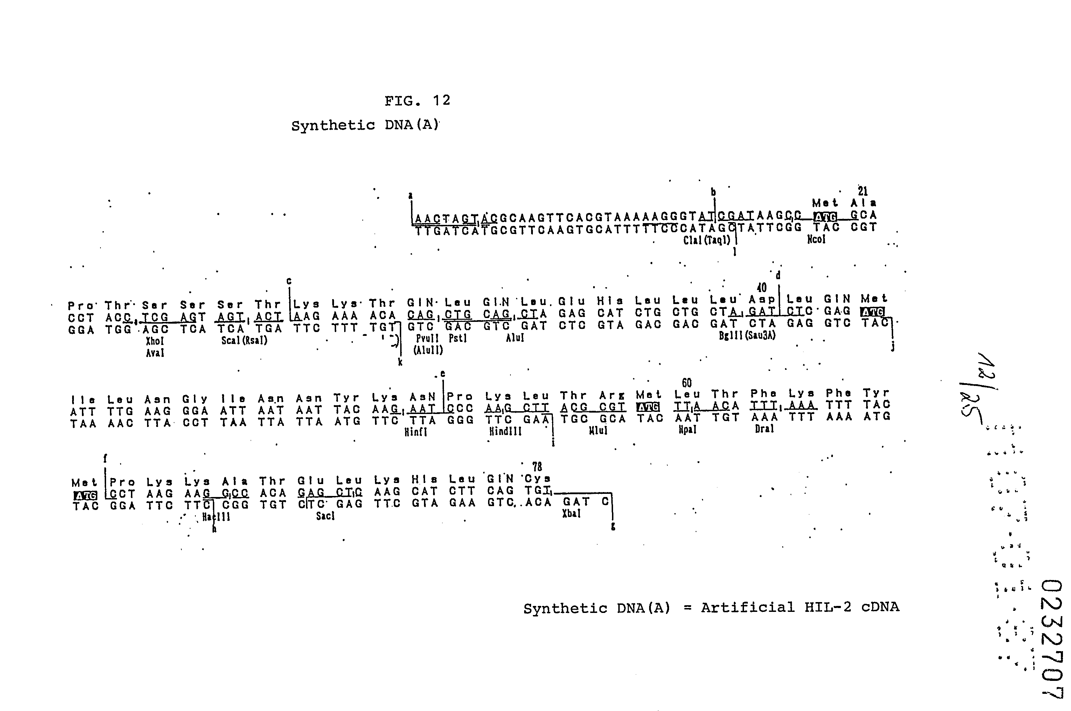

DNA Fragments (a) through (1) having the DNA sequence shown in Figure 12 were synthesized by the solid phase phosphoric acid triester method, respectively. The DNA fragments other than (a) and (g) were phosphorylated at the 5'-end thereof using T4 polynucleotide kinase and ATP.

Next, the DNA fragments (a) to (l) were mixed and annealed.Then using T4 DNA ligase, synthetic double stranded DNA (A ) was produced. On the other hand, pT9-11 (Japanese Unexamined Patent Publication 61-88882) was cleaved with restriction enzymes Hpa I and Xba I and large DNA fragments were separated by agarose gel electrophoresis. Next the obtained pT9-11 fragment was mixed with synthetic DNA (A) and the mixture was ligated using T4 DNA ligase. The obtained recombinant DNA was introduced into Escherichia coli HB 101 strains and a strain having ampicillin resistance was selected. From the separated strain plasmid DNA was obtained. By cleavage with restriction enzyme and determination of base sequence, bacteria harboring pT13S(Nco) were selected.

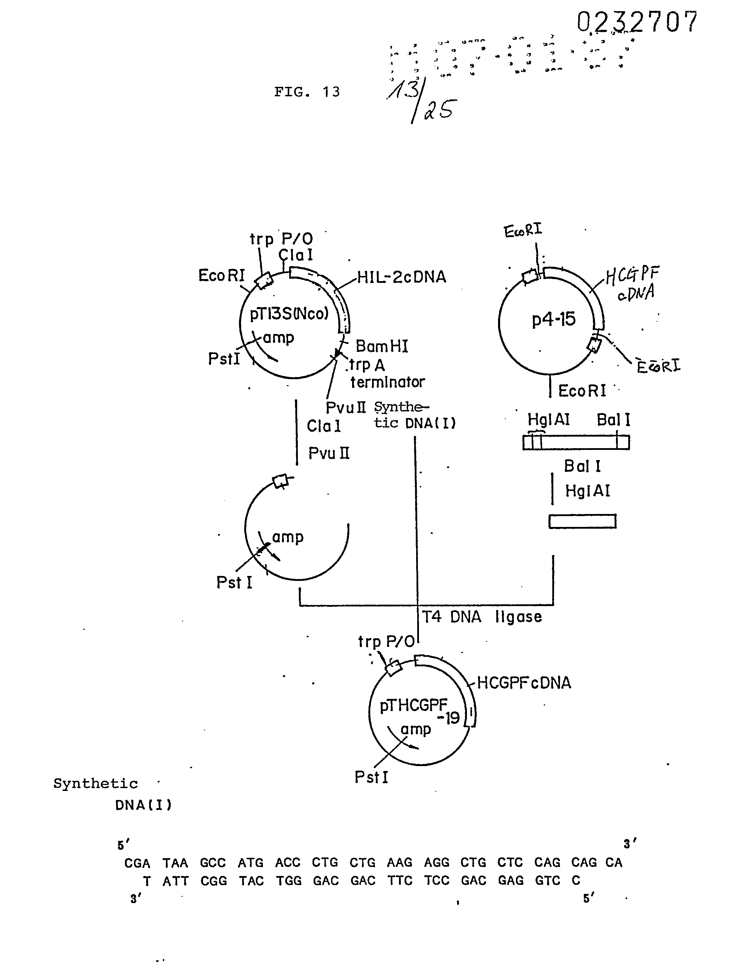

(2) Using plasmids pT13S(Nco) and p4-15, recombinant DNA for expressing HCGPF having Thr$^{19}$ at the N-terminal thereof was constructed as follows (Figure 13).

i) Plasmid pT13S(Nco) was cleaved with restriction enzymes Cla I and Pvu II and large DNA fragment was isolated and purified by agarose gel electrophoresis. On the other hand, plasmid p4-15 was cleaved with restriction enzyme EcoR I and HCGPF cDNA insert was recovered by agarose gel electrophoresis. The EcoRI HCGPF and cDNA insert were fully cleaved with restriction enzymes Bal I and HgiA I and, large DNA fragment was recovered by agarose gel electrophoresis. Next, pT13S(Nco) fragment containing tryptophan promoter/ operator (trp·P/O), HgiA I-Bal I fragment containing HCGPF cDNA and synthetic DNA (I) [$^{5'}$CGATAAGCCATGACCCTGCTGAAGA — GGCTGCTCCAGCAGCA$^{3'}$ and $^{5'}$CCTGGAGCAGCCTCTTCAGCAGGGTCATGGCTTATTAT$^{3'}$] were mixed and the mixture was ligated using T4 DNA ligase. The obtained recombinant DNA was introduced into Escherichia coli HB 101 strains and a strain having ampicillin resistance was selected. From the separated strain plasmid was prepared. By cleavage with restriction enzyme and determination of base sequence around the ligated site, bacteria harboring pTHCGPF-19 were selected (pTHCGPF-19/HB 101).

ii) pTHCGPF-19/HB 101 was grown at 37°C overnight in 10 ml of LG medium (1% bactotryptone, 0.5% yeast extract, 0.5% NaCl and 0.1% glucose, pH 7.5) containing 25 µg/ml of streptomycin and 25 µg/ml of ampicillin. Then 5 ml of the culture suspension was inoculated on M9-Casamino acid medium (0.6% $Na_2HPO_4 \cdot 12H_2O$, 0.3% $KH_2PO_4$, 0.05% NaCl, 0.1% $NH_4Cl$, 0.05% $MgSO_4 \cdot 7H_2O$, 0.00147% $CaCl_2$, 0.2% glucose, 0.2% Casamino acid, 0.02% L-leucine, 0.02% L-proline and 0.0002% thiamine hydrochloride, pH 7.4) followed by culturing at 28°C for 3 hours. Then 3-indolacrylic acid (IAA) was added to the system in a concentration of 25 µg/ml followed by inductive incubation at 23°C for 21 hours. The cultured cells were centrifuged and washed with 20 mM Tris-hydrochloride (pH 7.5, containing 30 mM NaCl) and then suspended in 8 ml of the same buffer. Thus protein produced in the cells was extracted by treatment with 1% sodium dodecyl sulfate (SDS) in the presence of 50 mM of EDTA or digestion with 1 mg/ml of lysozyme and then sonic treatment (50 W, 30 seconds).

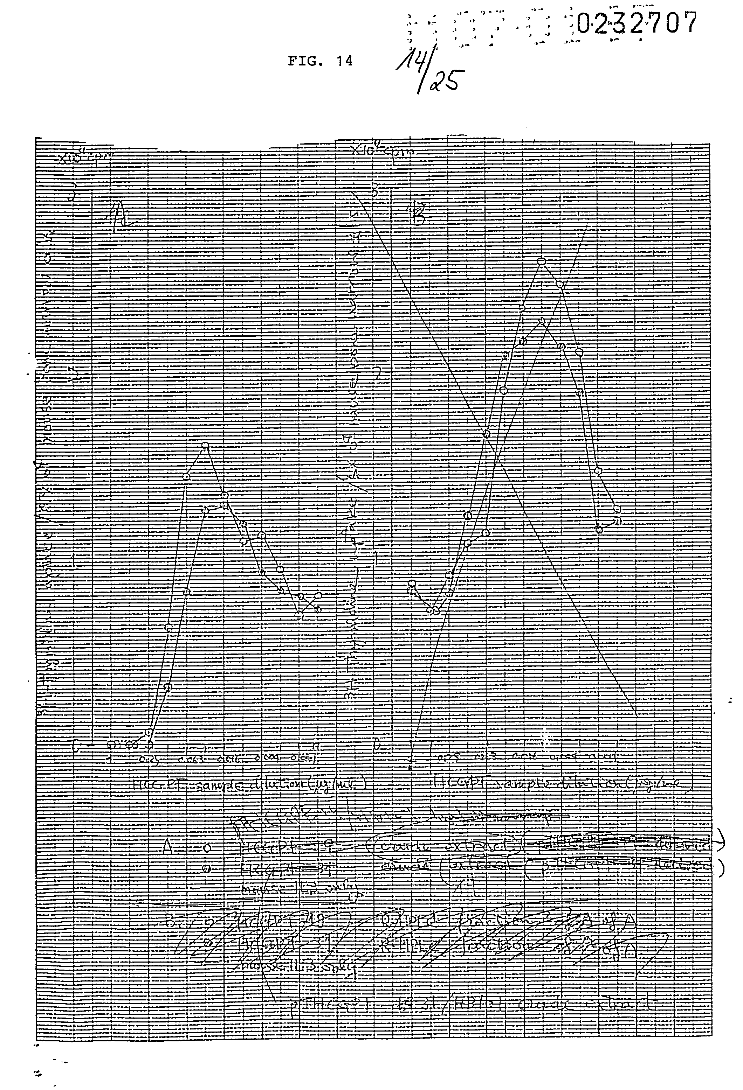

The culture extract showed the HCGPF activity (Fig. 14).

Example 5

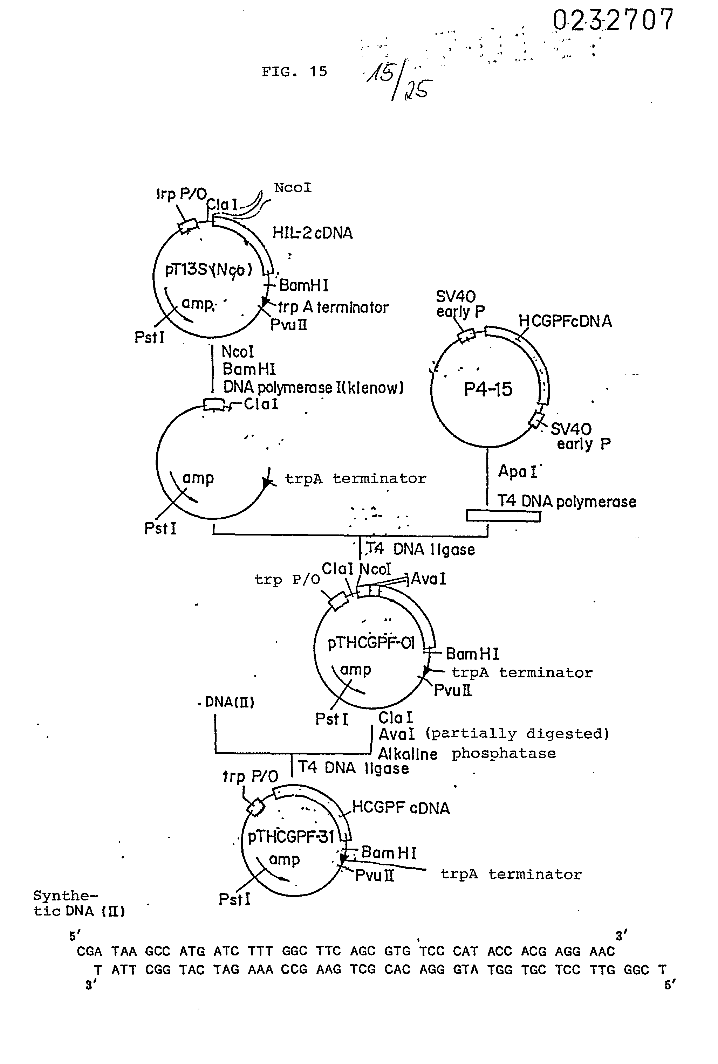

(1) Using plasmids pT13S(Nco) and p4-15, recombinant DNA for expressing HCGPF having $Ile^{31}$ at the N-terminal thereof was constructed as follows (Figure 15).

i) After plasmid pT13S(Nco) was cleaved with restriction enzymes Nco I and BamH I, the system was treated with DNA polymerase I (Klenow) and large DNA fragment was recovered by agarose gel electrophoresis. On the other hand, after plasmid p4-15 was cleaved with restriction enzyme Apa I, the system was treated with T4 DNA polymerase and small DNA fragment was recovered by agarose gel electrophoresis. These two fragments were ligated using T4 DNA ligase. The obtained recombinant DNA was introduced into Escherichia coli HB 101 strains and a strain having ampicillin resistance was selected. From the separated strain plasmid was obtained. By performing the cleavage test with restriction enzyme, bacteria harboring pTHCGPF-01 were selected (pTHCGPF-01/HB 101).

ii) After plasmid pTHCGPF-01 obtained in i) was fully cleaved with restriction enzyme Cla I, the system was partly cleaved with Ava I followed by treatment with alkaline phosphatase. The thus prepared DNA fragment and synthetic DNA (II) [$^{5'}$CGATAAGCCATGATCTTTGGCTTCAGCGTGT-CCCATACCACGAGGAAC$^{3'}$ and $^{5'}$TCGGGTTCCTCGTGGTATGGGACACGCTGAAG-CCAAAGATCATGGCTTAT$^{3'}$] were mixed and the mixture was ligated using T4 DNA ligase. The obtained recombinant DNA was introduced into Escherichia coli HB 101 strains and a strain having ampicillin resistance was selected. From the separated strain plasmid was obtained. By the cleavage test with restriction enzyme and determination of base sequence around the ligated site, bacteria harboring pTHCGPF-31 were selected (pTHCGPF-31/HB 101).

iii) pTHCGPF-31 /HB 101 was cultured in a manner similar to Example 4, ii) to give the cell extract. As shown in the figure 14, the culture extract showed the HCGPF activity.

Example 6

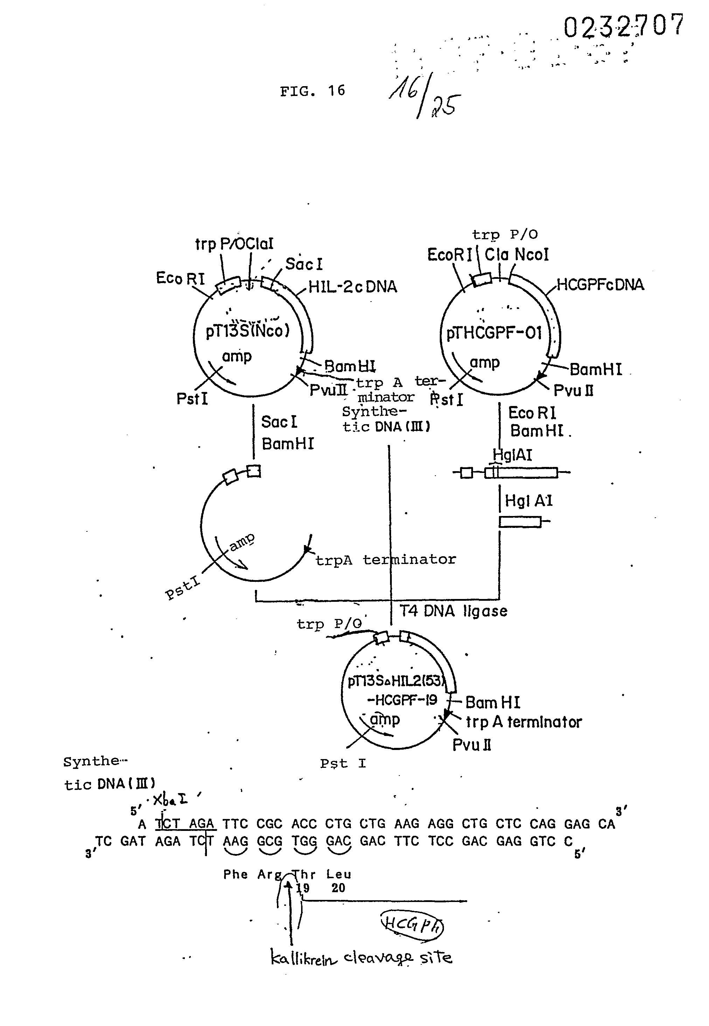

(1) Using plasmid pT13S(Nco) constructed in Example 4 and plasmid pTHCGPF-01 constructed in Example 5, recombinant DNA for expressing HCGPF having Thr$^{19}$ at the HIL-2-fused N-terminal thereof was constructed as follows (Figure 16).

i) After plasmid pT13S(Nco) was cleaved with restriction enzymes Sac I and BamH I, large DNA fragment was recovered by agarose gel electrophoresis. On the other hand, plasmid. pTHCGPF-01 was cleaved with restriction enzymes EcoR I and BamH.I and small DNA fragment was recovered by agarose gel electrophoresis. These DNA fragments were fully cleaved with restriction enzyme HgiA I and large DNA fragment was recovered by agarose gel electrophoresis. The thus prepared DNS fragment was mixed with synthetic DNS (III)

[$^5$' ATCTAGATTCCGCACCCTGCTGAAGA-

GGCTGCTCCAGGAGCA$^3$' and $^5$'CCTGGAGCAGCCTCTTCAGCA-

GGGTGCGGAATCTAGATAGCT$^3$' ] and the mixture

was ligated using T4 DNA ligase. The obtained recombinant DNA was introduced into <u>Escherichia</u> <u>coli</u> HB 101 strains and a strain having ampicillin resistance was selected. From the separated strain plasmid was obtained. By performing the cleavage test with restriction enzyme and determination of base sequence, bacteria harboring pTl3SΔHIL253-HCGPF-19 were selected (pTl3SΔHIL253-HCGP-19/HB 101).

(2) Harvest of the Product

pTl3SΔHIL2(53)-HCGPF-19/HB101 was cultured in a manner similar to Example 4 and granules produced in the cells were extracted by the following procedure.

The cells were collected by centrifugation and 20 mM of Tris-HCl buffer (pH 7.5) containing 30 mM of NaCl was added thereto followed by suspending them. Then 1 mg/ml of lysozyme and 0.05M of EDTA were added thereto. After stirring them, the mixture was allowed to stand in ice water for 1 hour. Then the cells were homogenized by a ultrasonic homogenizer and centrifuged at 10,000 rpm for 5 minutes to recover granules.

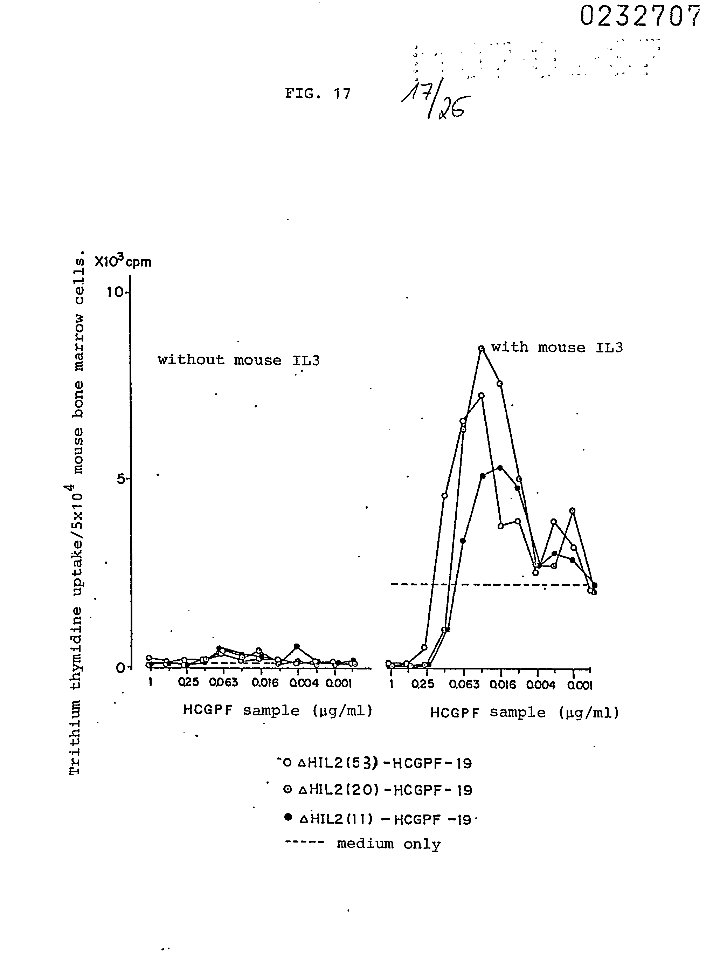

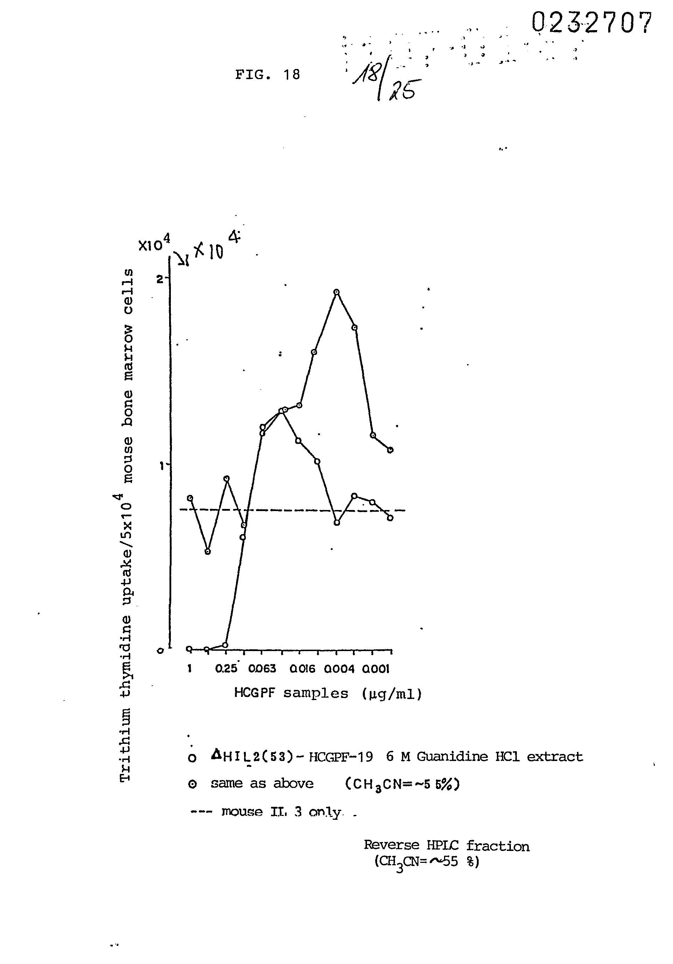

The granules were solubilized with 6M guanidine hydrochloride. As shown in the figure 17, the extract showed the HCGPF activity. Reversed phase HPLC fraction of the extract also showed the HCGPF activity (Figure 18). Concentrations of the extract were adjusted to give 100 µg/ml of HIL-2-(53)-HCGPF-protein and 2 M guanidine hydrochloride solution. Then 1 mM of oxidative glutathione and 10 mM of reductive glutathione were added thereto and the mixture was allowed to stand at room temperature for 10 to 16 hours at pH of 8.0. Guanidine hydroxhloride was removed by gel filtration with Sephadex G-25 and at the same time, a fraction corresponding to HIL-2-(53)-HCGPF-protein was obtained as a buffer solution for reacting with kallikrein. A molecular weight of the product obtained by SDS polyacrylamide gel electrophoresis was almost identical with one calculated from its amino acid composition. As a result of assay for amino acid sequence at the N-terminal side thereof with a protein sequencer, it was further confirmed that the product had a sequence of HIL-2.

(3) Cleavage with Kallikrein

After 80 µg of ΔHIL-2-53-HCGPF-19 protein obtained in 50 mM of Tris-HCl buffer (pH 7.8) containing 113 mM of NaCl was reacted with human plasma kallikrein at 37°C for 15 hours, a fraction corresponding to HCGPF-19 was fractionated by reversed phase HPLC. As a result of analysis of amino acid sequence around the N-terminal side thereof with a protein sequencer, it was confirmed that $\Delta$HIL-2-53-HCGPF-19 protein was quantitatively converted into HCGPF-19 protein.

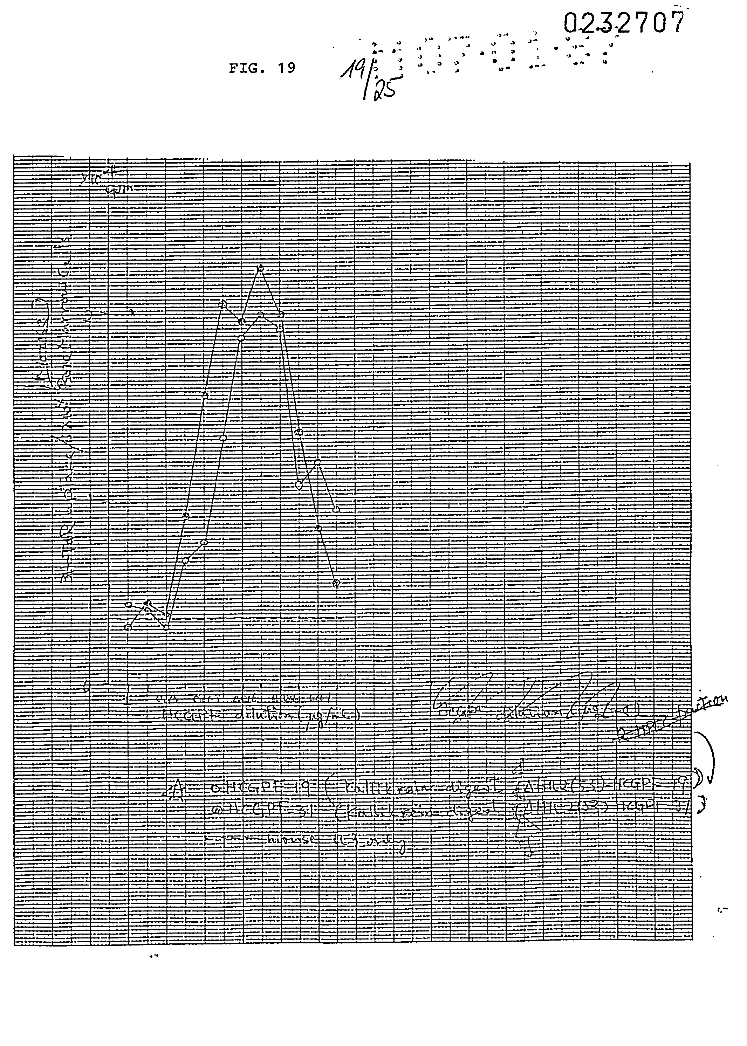

As shown in the figure 19, HCGPF-19 protein showed the HCGPF activity.

Example  7

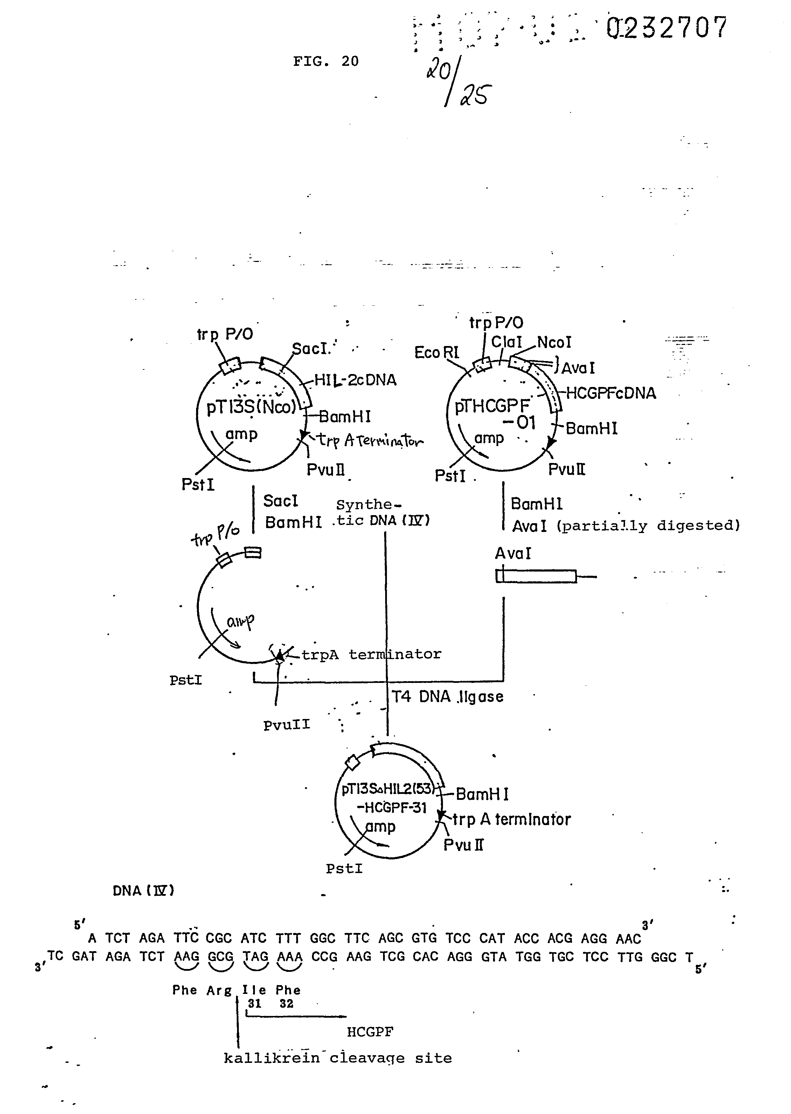

(1)  Using plasmids pT13S(Nco) and  pTHCGPF obtained in Examples  4 and 5, respectively, recombinant DNA for expressing HCGPF having Ile$^{31}$ at the HIL-2 protein-fused N-terminal thereof was constructed as follows (Figure 20).

i)  After plasmid pT13S(Nco) was cleaved with restriction enzyme Sac I and BamH I, large DNA fragment was recovered by agarose gel electrophoresis.  On the other hand, after plasmid  pTHCGPF-01 was fully cleaved with restriction enzyme BamH I, the system was partly cleaved with restriction enzyme Ava I and a DNA fragment containing HCGPF cDNA was recovered by agarose gel electrophoresis.  The thus prepared DNA fragments were mixed with synthetic DNA (IV) [$^{5'}$ ATC TAGATTCCGCATCTTTGGCTTCAGCGTGTCCCATACCACGAG AAC$^{3'}$ and $^{5'}$ TCG GGTTCCTCGTGGTATGGGACACGCTGAAGCCAAAGATGCGGAATCTAGATA GCT$^{3'}$] and the mixture was ligated with T4 DNA ligase. The obtained recombinant DNA was introduced into Escherichia coli HB 101 strains and a strain having ampicillin resistance was selected. From the separated strain plasmid was obtained. By performing the cleavage test with restriction enzyme and determination of base sequence, bacteria harboring pT13SΔHIL2-53-HCGPF-31 were selected (pT13SΔHIL2-53-HCGPF-31/HB 101).

(2) Harvest of the Product

pT13SΔHIL2(53)-HCGPF-31/HB 101 was cultured in a manner similar to Example 4 and granules produced in the cells were extracted in a manner similar to Example 6.

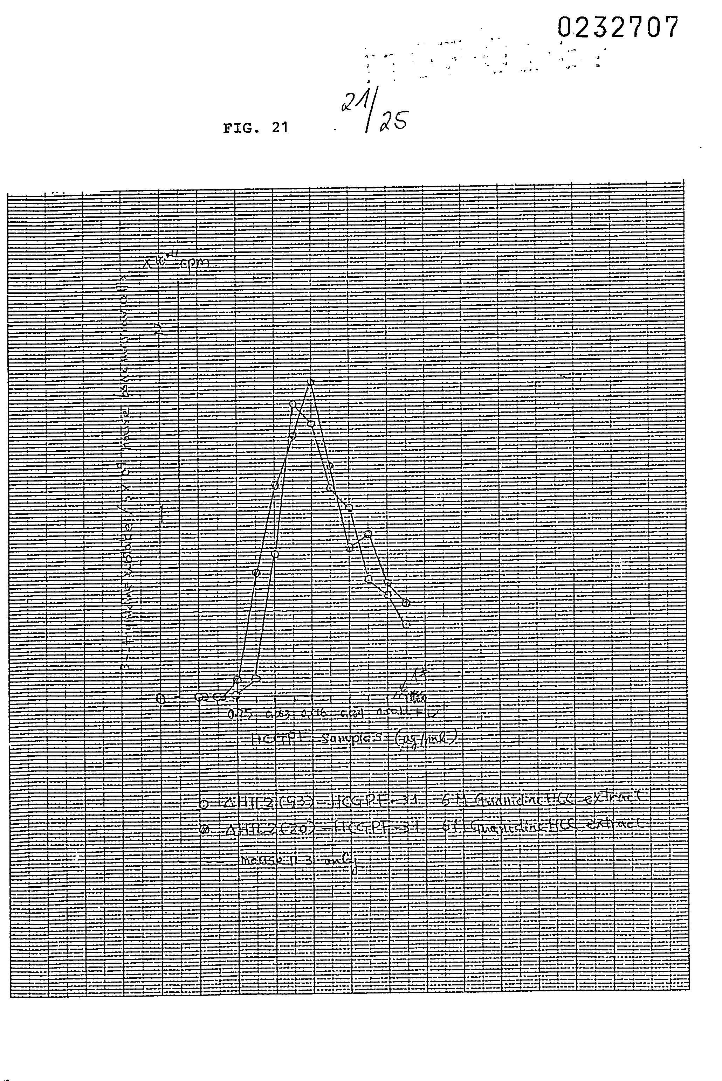

The granules were solubilized with 6M guanidine hydrochloride. As shown in the figure 21, the extract showed the HCGPF activity. Concentration of the extract was adjusted to give 100 µg/ml of ΔHIL-2-(53)-HCGPF-31 protein and 2 M guanidine hydrochloride solution. Then 1 mM of oxidative glutathione and 10 mM of reductive glutathione were added thereto and the mixture was allowed to stand at room temperature for 10 to 16 hours at pH of 8.0. Guanidine hydrochloride was removed by gel filtration with Sephadex G-25 and at the same time, a fraction corresponding to ΔHIL-2-(53)-HCGPF-31 protein was obtained as a buffer solution for reacting with kallikrein. The molecular weight of the product obtained by SDS polyacrylamide gel electrophoresis was almost identical with one calculated from its amino acid composition. As a result of assay for amino acid sequence at the N-terminal side thereof with a protein sequencer, it was further confirmed that the product had a sequence of HIL-2.

(3) Cleavage with Kallikrein

After 80 µg of ΔHIL-2-53-HCGPF-31 protein obtained in 50 mM of Tris-HCl buffer (pH 7.8) containing 113 mM of NaCl was reacted with human plasma kallikrein at 37°C for 15 hours, a fraction corresponding to HCGPF-31 protein was fractionated by reversed phase HPLC. As a result of analysis of amino acid sequence around the N-terminal side thereof with a protein sequencer, it was confirmed that ΔHIL-2-53-HCGPF-31 protein was quantitatively converted into HCGPF-31 protein. As shown in the figure 19 HCGPF-31 protein showed the HCGPF activity.

Example 8

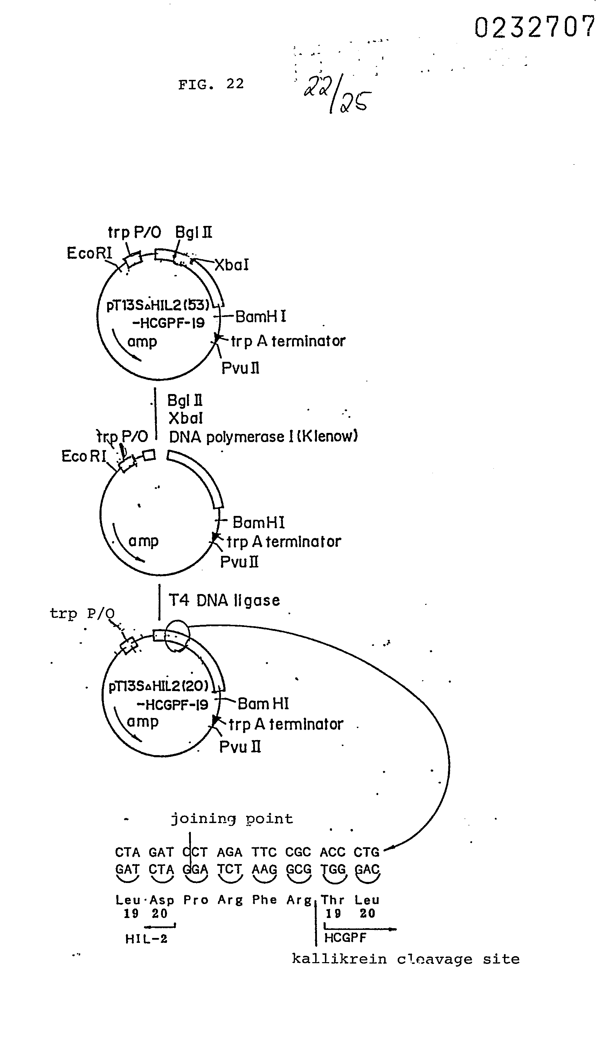

(1) i) Plasmid pT13ΔHIL2(20)-HCGPF-19 constructed in Example 6 was cleaved with restriction enzymes Bgl II and Xba I. After treating with DNA polymerase I (Klenow), the system was ligated with T4 DNA ligase (Figure 22). The obtained recombinant DNA was introduced into Escherichia coli HB 101 strains and a strain having ampicillin resistance was selected. From the separated strain plasmid DNA was obtained. By performing the cleavage test with restriction enzyme and determination of base sequence around the ligated site thereof, bacteria harboring pT13ΔHIL2(20)-HCGPF-19 were selected. (pT13ΔHIL2(20)- HCGPF-19/HB 101).

(2) Harvest of the Product

pT13SΔHIL2(20)-HCGPF-19/HB101 was cultured in a manner similar to Example 4 and granules produced in the cells were extracted by the procedures described in Examples 4 and 6.

As shown in the figure 17, the extract showed the HCGPF activity.

Concentrations of the extract were adjusted to give 100 μg/ml of ΔHIL2(20)-HCGPF-19 protein and 2 M guanidine hydrochloride solution. Then 1 mM of oxidative glutathione and 10 mM of reductive glutathione were added thereto and the mixture was allowed to stand at room temperature for 10 to 16 hours at pH of 8.0. Guanidine hydrochloride was removed by gel filtration with Sephadex G-25 and at the same time, a fraction corresponding to ΔHIL2-(20)-HCGPF-19 protein was obtained as a buffer solution for reacting with kallikrein. A molecular weight of the product obtained by SDS polyacrylamide gel electrophoresis was almost identical with one calculated from its amino acid composition. As a result of assay for amino acid sequence at the N-terminal side thereof with a protein sequencer, it was further confirmed that the product had a sequence of HIL-2.

(3) Cleavage with Kallikrein

After 80 μg of ΔHIL2(20)-HCGPF-19 protein obtained in 50 mM of Tris HCl buffer (pH 7.8) containing 113 mM of NaCl was reacted with human plasma kallikrein at 37°C for 15 hours, a fraction corresponding to HCGPF-19 protein was fractionated by reversed phase HPLC. As a result of analysis of amino acid sequence around the N-terminal side thereof with a protein sequencer, it was confirmed that ΔHIL2-(20)-HCGPF-19 protein was quantitatively converted into HCGPF-19 protein. The HCGPF-19 protein obtained showed the HCGPF activity.

Example 9

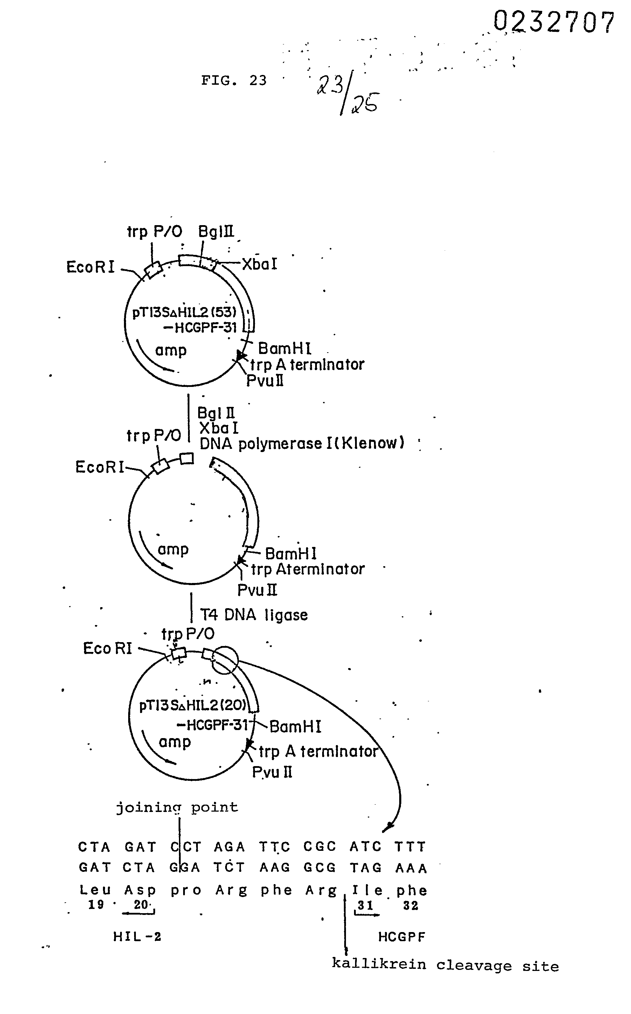

(1) i) Plasmid pTl3ΔHIL2(53)-HCGPF-31 constructed in Example 7 was cleaved with restriction enzymes Bgl II and Xba I. After treating with DNA polymerase I (Klenow), the system was ligated using T4 DNA ligase (Figure 23 ). The

0232707

- 44 -

obtained recombinant DNA was introduced into <u>Escherichia</u> <u>coli</u> HB 101 strains and a strain having ampicillin resistance was selected. From the separated strain plasmid DNA was obtained. By performing the cleavage test with restriction enzyme and determination of base sequence around the ligated site thereof, bacteria harboring pTl3△HIL2(20)-HCGPF-31 were selected (pTl3△HIL2(20)- HCGPF-31/HB 101).

(2) Harvest of the Product

pTl3S△HIL2(20)-HCGPF-31/HB101 was cultured and granules produced in the cells were extracted by the procedures described in Example 4 and 6.

As shown in the figure 17, the extract showed the HCGPF activity.

Concentrations of the extract were adjusted to give 100 µg/ml of ΔHIL-2(20)-HCGPF-31 protein and 2M guanidine hydrochloride solution. Then 1 mM of oxidative glutathione and 10 mM of reductive glutathione were added thereto and the mixture was allowed to stand at room temperature for 10 to 16 hours at pH of 8.0. Guanidine hydrochloride was removed by gel filtration with Sephadex G-25 and at the same time, a fraction corresponding to ΔHIL-2(20)-HCGPF-31 protein was obtained as a buffer solution for reacting with kallikrein. A molecular weight of the product obtained by SDS polyacrylamide gel electrophoresis was almost identical with one calculated from its amino acid composition. As a result of assay for amino acid sequence at the N-terminal side thereof with a protein sequencer, it was further confirmed that the product had a sequence of HIL-2.

(3) Cleavage with Kallikrein

After 80 µg of ΔHIL-2(20)-HCGPF-31 protein obtained in 50 mM of Tris-HCl buffer (pH 7.8) containing 113 mM of NaCl was reacted with human plasma kallikrein at 37°C for 15 hours, a fraction corresponding to HCGPF-31 protein was fractionated by reversed phase HPLC. As a result of analysis of amino acid sequence around the N-terminal side thereof with a protein sequencer, it was confirmed that ΔHIL-2(20)-HCGPF-31 protein was quantitatively converted into HCGPF-31 protein. The HCGPF-31 protein obtained showed the HCGPF activity.

Example 10

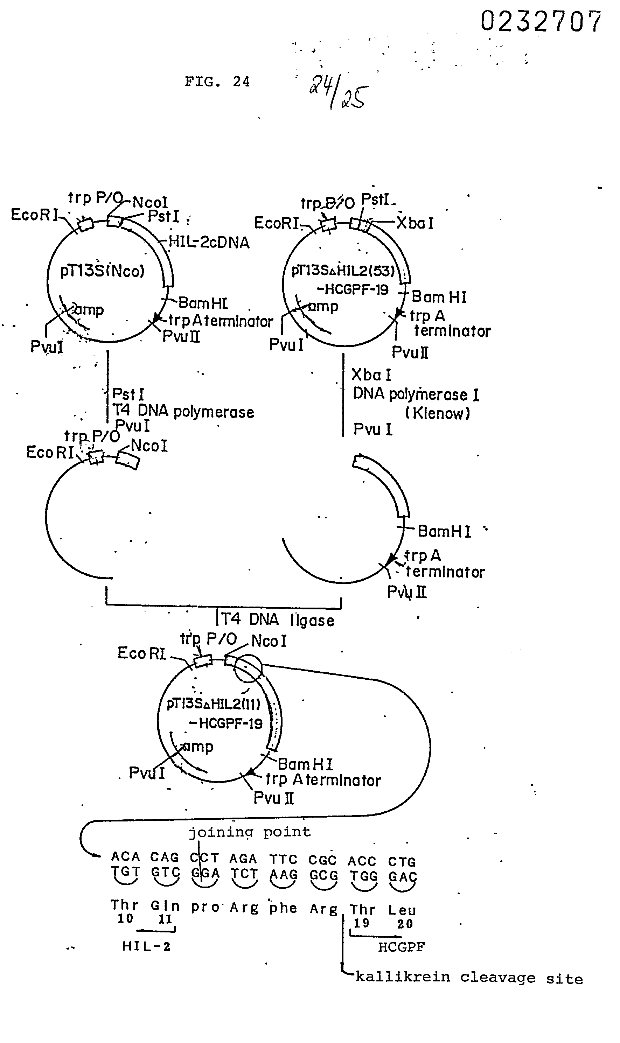

(1) Using plasmid pTl3S(Nco) constructed in Example 4 and plasmid pTl3S HIL2(53)-HCGPF-19 constructed in Example 6 recombinant DNA for expressing HCGPF having $Thr^{19}$ at the HIL-2 protein-fused N-terminal thereof was constructed as follows (Figure 24).

i) After plasmid pTl3S(Nco) was cleaved with restriction enzyme Pst I, the system was treated with T4 DNA polymerase. After further cleavage with restriction enzyme Pvu I, second large DNA fragment was recovered by agarose gel electrophoresis. On the other hand, after plasmid pTl3SΔHIL2(53)-HCGPF-19 was cleaved with restriction enzyme Xba I, the system was treated with DNA polymerase I (Klenow) and further cleaved with restriction enzyme Pvu I and, a large DNA fragment was recovered by agarose gel electrophoresis. These two fragments were ligated using T4 DNA ligase. The obtained recombinant DNA was introduced into Escherichia coli HB 101 strains and a strain having ampicillin resistance was selected. From the separated strain plasmid was obtained. By performing the cleavage test with restriction enzyme and determination of base sequence around the ligated site, bacteria harboring pTl3SΔHIL2(11)-HCGPF-19 were selected (pTl3SΔHIL2(11)-

HCGPF-19/HB 101).

(2) Harvest of the Product

PT13SΔHIL2(11)-HCGPF-19/HB101 was cultured in a manner similar to Example 4 and granules produced in the cells were extracted by the procedures described in Example 4 and 6.

As shown in the figure 17, the extract showed the HCGPF activity.

Concentrations of the extract were adjusted to give 100 μg/ml of ΔHIL2(11)-HCGPF-19 protein and 2 M guanidine hydrochloride solution. Then 1 mM of oxidative glutathione and 10 mM of reductive glutathione were added thereto and the mixture was allowed to stand at room temperature for 10 to 16 hours at pH of 8.0. Guanidine hydrochloride was removed by gel filtration with Sephadex G-25 and at the same time, a fraction corresponding to ΔHIL2(11)-HCGPF-19 protein was obtained as a buffer solution for reacting with kallikrein. The molecular weight of the product obtained by SDS polyacrylamide gel electrophoresis was almost identical with one calculated from its amino acid composition. As a result of assay for amino acid sequence at the N-terminal side thereof with a protein sequencer, it was further confirmed that the product had a sequence of HIL-2.

(3) Cleavage with Kallikrein

After 80 µg of ΔHIL2(11)-HCGPF-19 protein obtained in 50 mM of Tris-HCl buffer (pH 7.8) containing 113 mM of NaCl was reacted with human plasma kallikrein at 37°C for 15 hours; a fraction corresponding to HCGPF-19 protein was fractionated by reversed phase HPLC. As a result of analysis of amino acid sequence around the N-terminal side thereof with a protein sequencer, it was confirmed that ΔHIL-2-(11)-HCGPF-19 protein was quantitatively converted into HCGPF-19 protein. The HCGPF-19 protein obtained showed the HCGPF activity.

Example 11

Assay for HCGPF activity using mouse bone marrow cell:

(1) COS cell culture supernatant containing human HCGPF was prepared as follows. Plasmid pKCR 4-15, 5 µg, obtained in Example 3 was transfected with monkey COS-7 cells in accordance with the method of Example 2 to give the culture supernatant. The supernatant was concentrated by 5 times using ultrafiltration membrane (YM10) manufactured by Amicon Co., Ltd. to make a sample for measurement of human HCGPF activity.

(2) Preparation of the <u>Escherichia</u> <u>coli</u> extract containing human HCGPF and cleavage with kallikrein were carried out as in Example 6 to 10.

Culture extract, 6M guanidine hydrochloride extract, its fraction purified by reversed phase HPLC (AP-312, Yamamura Chemical) and kallikrein cleavage product were diluted in RPMI-1640 medium containing 20 % FBS, respectively, in a concentration of 1 μg/ml and the dilutions were made samples for measurement of human HCGPF activity.

(3) Assay

Assay for human HCGPF using mouse bone marrow cells was carried out as follows.

From DBA/2 female mice of 5 to 9 week age (Charles River Japan K.K.) the femur was withdrawn. After the both ends of the bone were ectomized, 1 ml of RPMI-1640 medium containing 10 % (volume ratio) FBS was injected from one side through a needle attached to a syringe thereby to exclude bone marrow cells in a plastic tube. Further a suspension of the bone marrow cells was suctioned and blown off through a needle attached to a syringe. By repeating the suction and blowoff, the cells were rendered single. After washing twice with RPMI-1640 medium containing 10 % FBS, the cells were suspended in RPMI-1640 medium containing 20 % FBS in a concentration of 1 x $10^6$/ml.

Samples to be measured were charged in each well of a 96 well tissue culture microplate by 100 μl each and 2-fold dilution was repeated with RPMI-1640 medium containing 20 % FBS. Then, the culture supernatant containing mouse interleukin 3 (IL3) obtained by transfection of 5 μg of plasmid pQRMIL3 containing mouse IL3 cDNA to monkey COS cells was added to[ each cell by 50 μl each.

Lastly, the thus prepared mouse bone marrow cell suspension was added to each well by 50 μl each. After incubation at 37°C for 5 days in the presence of 5% $CO_2$, 1 μCi of tritium thymidine was added. After pulsing for 8 hours, ß-radiations incorporated into the cells were counted in a conventional manner.

In the case of measuring direct stimulation of proliferation of HCGPF against bone marrow cells, 50 μl of RPMI-1640 medium containing 20 % FBS was added instead of the culture supernatant.

Among bone marrow cells, cells which grow in response to HCGPF can incorporate tritium thymidine in large amounts. Therefore, the amounts produced in the COS cell supernatant, the Escherichia coli extract, etc. can easily be determined quantitatively.

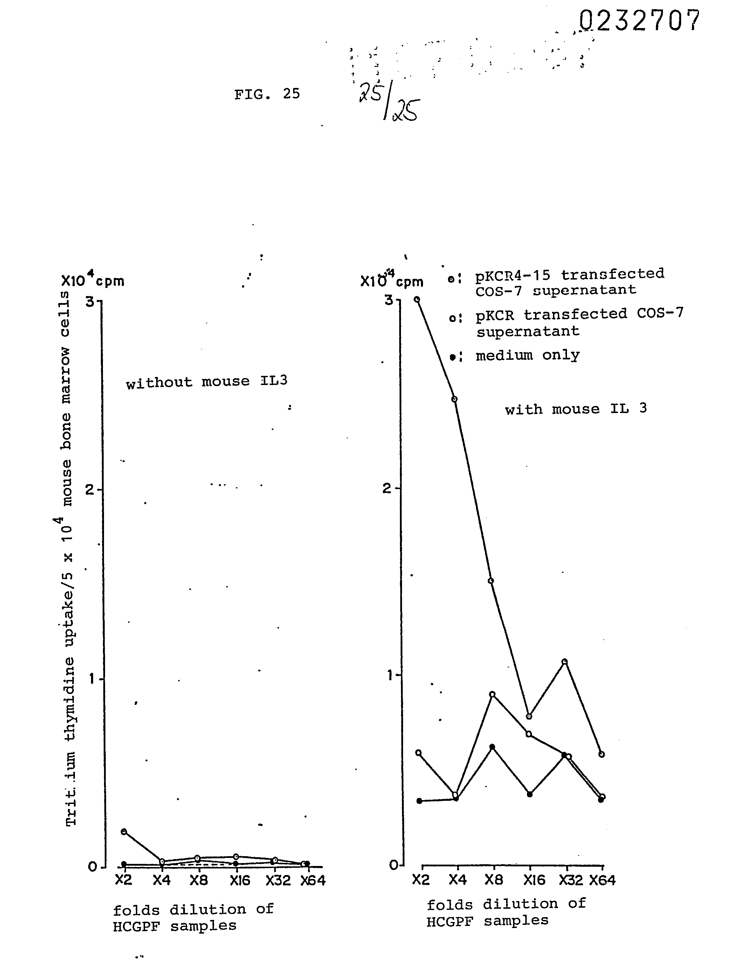

As shown in Figure 25, this method reveals that the COS supernatant contains the activity of proliferating bone marrow cells singly and the activity of proliferating bone marrow cells in combination with mouse IL3. None of the activities is recognized in the COS-7 cell supernatant obtained by transfection of cDNA-free plasmid pKCR used for control.

Culture extract of Escherichia coli, 6 M guanidine-HCl extract, its fraction purified by reversed phase HPLC and kallikrein cleavage product also showed the HCGPF activity (Figure 14, 17, 18, 19, 21).

Example 12

Assay using human bone marrow cells and thymocytes :

The HCGPF activity was measured using the same samples as used in Example 11 by the following method.

A suspension of human bone marrow cells ($2 \times 10^5$/well) or thymocytes ($5 \times 10^5$/well) obtained from volunteers in RPMI-1640 medium containing 10% FBS was subjected to double dilution. A sample obtained by the dilution, with which the HCGPF activity was to be measured, was charged in each well of a 96 well tissue culture plate by 100 µl each. After incubation at 37°C for 5 days in 5% $CO_2$, 1 µCi of tritium thymidine was added to each well followed by pulsing for 8 hours. The cells were harvested in a conventional manner and then, ß-radiation activity incorporated was determined. As shown in Table 2, significant induction in proliferation was noted although it was weak.

- 53 -

<center>Table 2</center>

| A. Sample | Amount of Tritium Thymidine Incorporated in Thymocyte* (cpm) | | | |
|---|---|---|---|---|
| | x2 | x4 | x8 | x16 |
| Transfected CQS-7 culture supernatant of pKCR | 1151 | 1232 | 548 | 1002 |
| Transfected COS-7 culture supernatant of pKCR-4-15 | 1550 | 2009 | 1066 | 524 |
| 6M Guanidine hydrochloride extract of Escherichia coli containing the following plasmid | x400 | x800 | x1600 | x3200 |
| Plasmid containing mouse IL2 cDNA | 224 | 395 | 505 | 240 |
| pT13SΔHIL2(20)-HCGPF-19 | 563 | 1266 | 2355 | 2749 |

\* mean value of 2 wells

|  | Amount of Tritium Thymidine Incorporated in Bone Marrow Cells* (cpm) | | | |
| Sample | x4 | x8 | x16 | x32 |
| Transfected COS-7 culture supernatant of pKCR | 3484 | 2605 | 4789 | 2505 |
| Transfected COS-7 culture supernatant of pKCR-4-15 | 4718 | 3394 | 5821 | 2450 |
| 6M Guanidine hydrochloride extract of Escherichia coli containing the following plasmid | x400 | x800 | x1600 | |
| Plasmid containing mouse IL2 cDNA | 672 | 1314 | 431 | |
| pT13SΔHIL2(20)-HCGPF-19 | 2861 | 1384 | 905 | |

\*   Mean value of 2 wells

Brief Description of the Drawings

Figure 1 is a cleavage map of cDNA insert with restriction enzyme endonuclease.

Figure 2 shows a base sequence of HCGPF.

Figure 3 shows amino acid sequence (I) of HCGPF deduced from cDNA sequence.

Figure 4 shows amino acid sequence (II) of HCGPF deduced from cDNA sequence.

Figure 5 shows amino acid sequence (III) of HCGPF deduced from cDNA sequence.

Figure 6 is a construction procedure of expression vector pDE-2.

Figure 7 shows FDC-P activity of the culture supernatant of COS cells in which p4-15 has been introduced, wherein:

□-□ p4-15 clone (concentrated by 4 times)

o-o p4-15 clone (undiluted)

●-● control (COS supernatant in which no gene has been introduced)

--- medium for activity assay

Figure 8 shows amino acid sequence of HCGPF deduced from cDNA.

Figure 9 shows amino acid sequence of HCGPF deduced from cDNA.

Figure 10 is a construction procedure of plasmid pKCR-4-15.

Figure 11 is a construction procedure of vector pT13S(Nco).

Figure 12 shows DNA fragment of human IL2.

Figure 13 is a construction procedure of plasmid pTHCGPF-19.

Figure 14 shows the HCGPF activity of crude extract of E. coli harboring various plasmids.

Figure 15 is a construction procedure of plasmid pTHCGPF-31.

Figure 16 is a construction of plasmid pT13SΔHIL2(53)-HCGPF-19.

- 56 -

Figure 17 shows the HCGPF activity of 6 M guanidine·HCl extracts of granules from E. coli harboring various plasmids with or without addition of mouse IL3.

Figure 18 shows the HCGPF activity of the reversed phase HPLC fraction of △HIL2(53)-HCGPF-19 protein.

Figure 19 shows the HCGPF activity of kallikrein-digests of human IL2-fused proteins.

**Figure 20 is a construction procedure of plasmid pT13S △HIL2(53)-HCGPF-31.**

Figure 21 shows the HCGPF activity of 6 M guanidine HCl extract of granules from E. coli harboring various plasmids.

Figure 22 is a construction procedure of plasmid pT13S △HIL2-(20)-HCGPF-19.

Figure 23 is a construction procedure of plasmid pT13S△HIL2-(20)-HCGPF-31.

Figure 24 is a construction procedure of plasmid pT13S △HIL2-(11)-HCGPF-19.

Figure 25 shows the HCGPF activity of the COS cell culture supernatant with or without addition of mouse IL 3.

CLAIMS

1. A gene coding for a polypeptide having human hematopoietic cell growth potentiating factor (HCGPF) activity .

2. A gene as claimed in claim 1 wherein said gene contains a portion in which sites cleaved with restriction endonuclease are arranged in the order of Sph I, Pst I and Ban I from the 5'-end of the coding sequence.

3. A gene as claimed in claim 1 wherein said gene contains a portion in which sites cleaved with restriction endonuclease are arranged in the order of Ava I, Sph I, Ava I, Pst I, Ban I and BstE II from the 5'-end of the coding sequence. ·

4. A gene as claimed in claim 1 wherein said gene has the following base sequence (a):

Base Sequence (a):

1 *                                                                    * 60
GGA.TGC.TGC.GGC.GCC.CGC.TGG.CCG.GGC.GGC.TGC.GGC.CGC.CCT.GGC.CGG.GCC.CCA.CCG.GAC

61 *                                                                    * 120
GGC.ATG.TCG.GGC.CCC.AGG.CCT.GTG.GTG.CTG.AGC.GGG.CCT.TCG.GGA.GCT.GGG.AAG.AGC/ACC

121 *                                                                   * 180
CTG.CTG.AAG.AGG.CTG.CTC.CAG.GAG.CAC.AGC.GGC.ATC.|TTT.GGC.TTC.AGC.GTG.TCC.CAT.ACC

181 *                                                                   * 240
ACG.AGG.AAC.CCG.AGG.CCC.GGC.GAG.GAG.AAC.GGC.AAA.GAT.TAC.TAC.TTT.GTA.ACC.AGG.GAG

241 *                                                                   * 300
GTG.ATG.CAG.CGT.GAC.ATA.GCA.GCC.GGC.GAC.TTC.ATC.GAG.CAT.GCC.GAG.TTC.TCG.GGG.AAC

301 *                                                                   * 360
CTG.TAT.GGC.ACG.AGC.AAG.GTG.GCG.GTG.CAG.GCC.GTG.CAG.GCC.ATG.AAC.CGC.ATC.TGT.GTG

361 *                                                                   * 420
CTG.GAC.GTG.GAC.CTG.CAG.GGT.GTG.CGG.AAC.ATC.AAG.GCC.ACC.GAT.CTG.CGG.CCC.ATC.TAC

421 *                                                                   * 480
ATC.TCT.GTG.CAG.CCG.CCT.TCA.CTG.CAC.GTG.CTG.GAG.CAG.CGG.CTG.CGG.CAG.CGC.AAC.ACT

481 *                                                                   * 540
GAA.ACC.GAG.GAG.AGC.CTG.GTG.AAG.CGG.CTG.GCT.GCT.GCC.CAG.GCC.GAC.ATG.GAG.AGC.AGC

541 *                                                                   * 600
AAG.GAG.CCC.GGC.CTG.TTT.GAT.GTG.GTC.ATC.ATT.AAC.GAC.AGC.CTG.GAC.CAG.GCC.TAC.GCA

601 *                                                                   * 660
GAG.CTG.AAG.GAG.GCG.CTC.TCT.GAG.GAA.ATC.AAG.AAA.GCT.CAA.AGG.ACC.GGC.GCC.TGA.GGC.

721 *                                                                   * 720
TTG.CTG.TCT.GTT.CTC.GGC.ACC.CTG.GGC.CCA.TAC.AGG.ACC.AGG.GCA.GCA.GCA.TTG.AGC.CAC

781 *                                                                   * 780
CCC.CCT.TGG.CAG.GCG.ATA.CGG.CAG.CTC.TGT.GCC.CTT.GGC.CAG.CAT.GTG.GAG.TGG.AGG.AGA

841 *                                                                   * 840
TGC.TGC.CCC.TGT.GGT.TGG.AAC.ATC.CTG.GGG.TGA.CCC.CCG.ACC.CAG.CCT.CGC.TGG.GCT.GTC

901 *                                                                   * 900
CCC.TGT.CCC.TAT.CTC.TCA.CTC.TGA.ACC.CAG.GGC.TGA.CAT.CCT.AAT.AAA.ATA.ACT.GTT.GGA

*
TTA.GAA.ACT.

5. A gene as claimed in claim 1 wherein said gene starts with ATG sequence at the 64- to 66-positions in said sequence (a) described in claim 4 and has a sequence space from said ATG sequence to GCC sequence at least at the 52- to 54-positions.

6. A gene as claimed in claim 1 wherein said gene starts with ACC sequence at the 118- to 120-positions in said sequence (a) described in claim 4 and has a sequence space from said ACC sequence to GCC sequence at least at the 52- to 54-positions.

7. A gene as claimed in claim 1 wherein said gene starts with ATC sequence at the 154- to 156-positions in said sequence (a) claimed in claim 4 and has a sequence space from said ATC sequence to GCC sequence at least at the 652- to 654-positions.

8. A gene as claimed. in claim 1 wherein said gene starts with ACC sequence at the 118- to 120-positions in said sequence (a) claimed in claim 4.`

9. A gene as claimed in claim 1 wherein said gene starts with ATC sequence at the 154- to 156-positions in said sequence (a) claimed in claim 4.

10. A gene as claimed in claim 1 wherein said gene ends with GCC sequence at the 652- to 654-positions in said base sequence (a) claimed in claim 4.

- 60 -

11. A gene as claimed in claim 1 wherein said gene has a base sequence corresponding to the following amino acid sequence (I):

Amino Acid Sequence (I):

Thr

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

12. A gene as claimed in claim 1 wherein said gene has a base sequence corresponding to the following amino acid sequence (II):

Amino Acid Sequence (II):

Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

13. A gene as claimed in claim 1 wherein said gene has a base sequence corresponding to the following amino acid sequence (III):

Amino Acid Sequence (III):

MET-Ser-Gly-Pro-Arg-Pro-Val-Val-Leu-Ser-Gly-Pro-Ser-Gly-Ala-Gly-Lys-Ser-Thr

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

14. A recombinant DNA comprising a gene conding for polypeptide having human HCGPF activity and a vector DNA capable of replicating in eucaryocytes or procaryocytes.

15. A recombinant DNA as claimed in claim 14 wherein said gene has the base sequence (a) claimed in claim 4.

16. A recombinant DNA as claimed in claim 14 wherein said gene is the gene claimed in any of the claims 5 to 13.

17 . A recombinant DNA as claimed in claim 14 wherein said procaryocyte is monkey cell transformed with SV-40 for constructurally expressing large T antigen.

18 . Eucaryocyte and procaryocyte transformed by the recombinant DNA of any of the claims 14 to 16.

19. The cell as claimed in claim 18, wherein said eucaryocyte is monkey cell transformed with SV-40 for constructurally expressing large T antigen.

20. The cell as claimed in claim 18, wherein said eucaryocyte belongs to the genus Saccharomyces.

21. The cell as claimed in claim 18, wherein said procaryocyte belongs to the genus Escherichia.

22. A method for the production of human HCGPF which comprises culturing a eucaryocyte or procaryocyte claimed in claim 18 in a medium and harvesting human HCGPF produced.

23. A method for production as claimed in claim 22, wherein said eucaryocyte belongs to the genus Saccharomyces.

24. A method for production as claimed in claim 22, wherein said eucaryocyte is monkey cells transformed with SV-40 for constructurally expressing large T antigen.

25. A method for production as claimed in claim 22, wherein said procaryocyte belongs to the genus *Escherichia*.

26. Polypeptide having human hemotopoietic cell growth potentiating factor (HCGPF) activity.

27. Polypeptide as claimed in claim 26, wherein said polypeptide has Thr at the N-terminal thereof.

28. Polypeptide as claimed in claim 26 wherein said polypeptide has Ile at the N-terminal thereof.

29. Polypeptide as claimed in claim 26 wherein said polypeptide has Ala at the C-terminal thereof.

30. Polypeptide as claimed in claim 26 wherein said polypeptide has Thr at the N-terminal thereof and Ala at the C-terminal thereof.

31. Polypeptide as claimed in claim 26 wherein said polypeptide has Ile at the N-terminal thereof and Ala at the C-terminal thereof.

32. Polypeptide as claimed in claim 26 wherein said polypeptide has the following amino acid sequence (I).

Amino Acid Sequence (I):

Thr

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

33. Polypeptide as claimed in claim 26 wherein said polypeptide has the following amino acid sequence (II).

Amino Acid Sequence (II):

Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

- 67 -

34 . Polypeptide as claimed in claim 26  wherein said polypeptide has the following amino acid sequence (III).

<u>Amino Acid Sequence (III):</u>

MET-Ser-Gly-Pro-Arg-Pro-Val-Val-Leu-Ser-Gly-Pro-Ser-Gly-Ala-Gly-Lys-Ser-Thr

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

- 68 -

35 . Polypeptide as claimed in claim 26 wherein said polypeptide has the following amino acid sequence (IV).

<u>Amino Acid Sequence (IV):</u>

Ala-Pro-Thr-Ser-Ser-Ser-Thr-Lys-Lys-Thr-Gln-Pro-Arg-Phe-Arg-Thr

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

36 . Polypeptide as claimed in claim 26 wherein said polypeptide has the following amino acid sequence (V).

Amino Acid Sequence (V):

Ala-Pro-Thr-Ser-Ser-Ser-Thr-Lys-Lys-Thr-Gln-Leu-Gln

Leu-Glu-His-Leu-Leu-Leu-Asp-Pro-Arg-Phe-Arg-Thr-

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

37 . Polypeptide as claimed in claim 26 wherein said polypeptide has the following amino acid sequence (VI).

Amino Acid Sequence (VI):

Ala-Pro-Thr-Ser-Ser-Ser-Thr-Lys-Lys-Thr-Gln-Leu-Gln

Leu-Glu-His-Leu-Leu-Leu-Asp-Leu-Gln-Met-Ile-Leu-Asn

Gly-Ile-Asn-Asn-Tyr-Lys-Asn-Pro-Lys-Leu-Thr-Arg-Met

Leu-Thr-Phe-Lys-Phe-Tyr-Met-Pro-Lys-Lys-Ala-Thr-Glu

Leu-Ser-Arg-Phe-Arg-Thr

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

38 . Polypeptide as claimed in claim 26 wherein said polypeptide has the following amino acid sequence (VII).

Amino Acid Sequence (VII):

Ala-Pro-Thr-Ser-Ser-Ser-Thr-Lys-Lys-Thr-Gln-Leu-Gln-Leu-Glu-His-Leu-Leu

Leu-Asp-Pro-Arg-Phe-Arg-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

0232707

- 72 -

39 . Polypeptide as claimed in claim 26 wherein said polypeptide has the following amino acid sequence (VIII).

Amino Acid Sequence (VIII):

Ala-Pro-Thr-Ser-Ser-Ser-Thr-Lys-Lys-Thr-Gln-Leu-Gln-

Leu-Glu-His-Leu-Leu-Leu-Asp-Leu-Gln-Met-Ile-Leu-Asn-

Gly-Ile-Asn-Asn-Tyr-Lys-Asn-Pro-Lys-Leu-Thr-Arg-Met

Leu-Thr-Phe-Lys-Phe-Tyr-Met-Pro-Lys-Lys-Ala-Thr-Glu-Leu

Ser-Arg-Phe-Arg-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-MET-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-MET-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-MET-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

40. Polypeptide as claimed in any of the claims 26 to 39 which has a structure wherein one or plural amino acid(s) in the amino acid sequences (I) through (VIII) is/are replaced by other amino acid(s) without loss of the activity.

41 . Polypeptide as claimed in claim 26 or 40 wherein said polypeptide is deficient in one or more amino acid(s) from from the N-terminal thereof or the C-terminal thereof among said amino acid sequences (I) through (VIII) and comprises a plurality of continuing amino acids.

42. Polypeptide as claimed in any of the claims 26 to 39, wherein one or more amino acid(s) is/are added to the N-terminal and/or C-terminal thereof in said amino acid sequences (I) through (VIII).

43 .. Polypeptide as claimed in any of the claims 26 to 42 which is produced using DNA recombined in procaryocyte or eucaryocyte.

44 . Polypeptide having human HCGPF activity comprising a sugar region produced in eukaryote wherein the polypeptide moiety is as defined in any of the claims 26 to 42.

1/25

FIG. 1

```
*   1                                                                    60*
GGA.TGC.TGC.GGC.GCC.CGC.TGG.CCG.GGC.GGC.TGC.GGC.CGC.CCT.GGC.CGG.GCC.CCA.CCG.GAC


*   61                                                                   120*
GGC.ATG.TCG.GGC.CCC.AGG.CCT.GTG.GTG.CTG.AGC.GGG.CCT.TCG.GGA.GCT.GGG.AAG.AGC.ACC
    MET-Ser-Gly-Pro-Arg-Pro-Val-Val-Leu-Ser-Gly-Pro-Ser-Gly-Ala-Gly-Lys-Ser-Thr


*   121                                                                  180*
CTG.CTG.AAG.AGG.CTG.CTC.CAG.GAG.CAC.AGC.GGC.ATC.TTT.GGC.TTC.AGC.GTG.TCC.CAT.ACC
Leu-Leu-Lys-Arg-Leu-Leu-Gln-Glu-His-Ser-Gly-Ile-Phe-Gly-Phe-Ser-Val-Ser-His-Thr


*   181                                                                  240*
ACG.AGG.AAC.CCG.AGG.CCC.GGC.GAG.GAG.AAC.GGC.AAA.GAT.TAC.TAC.TTT.GTA.ACC.AGG.GAG
Thr-Arg-Asn-Pro-Arg-Pro-Gly-Glu-Glu-Asn-Gly-Lys-Asp-Tyr-Tyr-Phe-Val-Thr-Arg-Glu


*   241                                                                  300*
GTG.ATG.CAG.CGT.GAC.ATA.GCA.GCC.GGC.GAC.TTC.ATC.GAG.CAT.GCC.GAG.TTC.TCG.GGG.AAC
Val-Met-Gln-Arg-Asp-Ile-Ala-Ala-Gly-Asp-Phe-Ile-Glu-His-Ala-Glu-Phe-Ser-Gly-Asn


*   301                                                                  360*
CTG.TAT.GGC.ACG.AGC.AAG.GTG.GCG.GTG.CAG.GCC.GTG.CAG.GCC.ATG.AAC.CGC.ATC.TGT.GTG
Leu-Tyr-Gly-Thr-Ser-Lys-Val-Ala-Val-Gln-Ala-Val-Gln-Ala-Met-Asn-Arg-Ile-Cys-Val


*   361                                                                  420*
CTG.GAC.GTG.GAC.CTG.CAG.GGT.GTG.CGG.AAC.ATC.AAG.GCC.ACC.GAT.CTG.CGG.CCC.ATC.TAC
Leu-Asp-Val-Asp-Leu-Gln-Gly-Val-Arg-Asn-Ile-Lys-Ala-Thr-Asp-Leu-Arg-Pro-Ile-Tyr


*   421                                                                  480*
ATC.TCT.GTG.CAG.CCG.CCT.TCA.CTG.CAC.GTG.CTG.GAG.CAG.CGG.CTG.CGG.CAG.CGC.AAC.ACT
Ile-Ser-Val-Gln-Pro-Pro-Ser-Leu-His-Val-Leu-Glu-Gln-Arg-Leu-Arg-Gln-Arg-Asn-Thr


*   481                                                                  540*
GAA.ACC.GAG.GAG.AGC.CTG.GTG.AAG.CGG.CTG.GCT.GCT.GCC.CAG.GCC.GAC.ATG.GAG.AGC.AGC
Glu-Thr-Glu-Glu-Ser-Leu-Val-Lys-Arg-Leu-Ala-Ala-Ala-Gln-Ala-Asp-Met-Glu-Ser-Ser


*   541                                                                  600*
AAG.GAG.CCC.GGC.CTG.TTT.GAT.GTG.GTC.ATC.ATT.AAC.GAC.AGC.CTG.GAC.CAG.GCC.TAC.GCA
Lys-Glu-Pro-Gly-Leu-Phe-Asp-Val-Val-Ile-Ile-Asn-Asp-Ser-Leu-Asp-Gln-Ala-Tyr-Ala


*   601                                                                  660*
GAG.CTG.AAG.GAG.GCG.CTC.TCT.GAG.GAA.ATC.AAG.AAA.GCT.CAA.AGG.ACC.GGC.GCC.TGA.GGC
Glu-Leu-Lys-Glu-Ala-Leu-Ser-Glu-Glu-Ile-Lys-Lys-Ala-Gln-Arg-Thr-Gly-Ala-***


*   661                                                                  720*
TTG.CTG.TCT.GTT.CTC.GGC.ACC.CTG.GGC.CCA.TAC.AGG.ACC.AGG.GCA.GCA.GCA.TTG.AGC.CAC


*   721                                                                  780*
CCC.CCT.TGG.CAG.GCG.ATA.CGG.CAG.CTC.TGT.GCC.CTT.GGC.CAG.CAT.GTG.GAG.TGG.AGG.AGA


*   781                                                                  840*
TGC.TGC.CCC.TGT.GGT.TGG.AAC.ATC.CTG.GGG.TGA.CCC.CCG.ACC.CAG.CCT.CGC.TGG.GCT.GTC


*   841                                                                  900*
CCC.TGT.CCC.TAT.CTC.TCA.CTC.TGA.ACC.CAG.GGC.TGA.CAT.CCT.AAT.AAA.ATA.ACT.GTT.GGA


*   901
TTA.GAA.ACT.
```

0232707

FIG. 3    3/25

Amino Acid Sequence (I)

Thr

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-LYS-Asp-Tyr-Tyr-Phe-Val-Thr-Arg-Glu

Val-Met-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-Met-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-Met-Glu-Ser-Ser

Lys-Glu-Pro-Gly-Leu-Phe-Asp-Val-Val-Ile-Ile-Asn-Asp-Ser-Leu-Asp-Gln-Ala-Thr-Ala

Glu-Leu-Lys-Glu-Ala-Leu-Ser-Glu-Glu-Ile-Lys-Lys-Ala-Gln-Arg-Thr-Gly-Ala

Amino Acid Sequence (II)

                                        Ile-Phe-Gly-Phe-Ser-Val-Ser-His-Thr

Thr-Arg-Asn-Pro-Arg-Pro-Gly-Glu-Glu-Asn-Gly-LYS-Asp-Tyr-Tyr-Phe-Val-Thr-Arg-Glu

Val-Met-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-Met-Asn- Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-Met-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg- Thr-Gly-Ala

Amino Acid Sequence (IV)

Met-Ser-Gly-Pro-Arg-Pro-Val-Val-Leu-Ser-Gly-Pro-Ser-Gly-Ala-Gly-Lys-Ser-Thr ·

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-Lys-Asp-Tyr-Tyr-Phe-Val-Thr-Arg-Glu

Val-Met-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val-Ala-Val-Gln-Ala-Val-Gln-Ala-Met-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-Met-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

FIG. 6    6/25    0232707

FIG. 7    7/25

FIG. 7

FIG. 8

MET-Ser-Gly-Pro-Arg-Pro-Val-Val-Leu-Ser-Gly-Pro-Ser-Gly-Ala-Gly-Lys-Ser-Thr

Leu-Leu-Lys-Arg-Leu-Leu-Gln-Glu-His-Ser-Gly-Ile-Phe-Gly-Phe-Ser-Val-Ser-His-Thr

Thr-Arg-Asn-Pro-Arg-Pro-Gly-Glu-Glu-Asn-Gly-LYS-Asp-Tyr-Tyr-Phe-Val-Thr-Arg-Glu

Val-Met-Gln-Arg-Asp-Ile-Ala-Ala-Gly-Asp-Phe-Ile-Glu-His-Ala-Glu-Phe-Ser-Gly-Asn

Leu-Tyr-Gly-Thr-Ser-Lys-Val- Ala-Val-Gln-Ala—Val-Gln-Ala-Met-Asn-Arg-Ile-Cys-Val

Leu-Asp-Val-Asp-Leu-Gln-Gly-Val-Arg-Asn-Ile-Lys-Ala-Thr-Asp-Leu-Arg-Pro-Ile-Tyr

Ile-Ser-Val-Gln-Pro-Pro-Ser-Leu-His-Val-Leu-Glu-Gln-Arg-Leu-Arg-Gln-Arg-Asn-Thr

Glu-Thr-Glu-Glu-Ser-Leu-Val-Lys-Arg-Leu-Ala-Ala-Ala-Gln-Ala-Asp-Met-Glu-Ser-Ser

Lys-Glu-Pro-Gly-Leu-Phe-Asp-Val-Val-Ile-Ile-Asn-Asp-Ser-Leu-Asp-Gln-Ala-Tyr-Ala

Glu-Leu-Lys-Glu-Ala-Leu-Ser-Glu-Glu-Ile-Lys-Lys-Ala-Gln-Arg-Thr-Gly-Ala

FIG. 9

Met-Leu-Arg-Arg-Pro-Leu-Ala-Gly-Arg-Leu-Arg-Pro-Pro-Trp-Pro-Gly-Pro-Thr-Gly-Arg

His-Val-Gly-Pro-Gln-Ala-Cys-Gly-Ala-Glu-Arg-Ala-Phe-Gly-Ser-Trp-Glu-Glu-His-Pro

Ala-Glu-Glu-Ala-Ala-Pro-Gly-Ala-Gln-Arg-His-Leu-Trp-Leu-Gln-Arg-Val-Pro-Tyr-His

Glu-Glu-Pro-Glu-Ala-Arg-Arg-Gly-Glu-Arg-Gln-Arg-Leu-Leu-Leu-Cys-Asn-Gln-Gly-Gly

Asp-Ala-Ala

FIG. 10

trp P/O
HpaI
EcoRI    XbaI
HIL-2cDNA
pT9-11
BamHI
ampP    trpA terminator
PvuII

HpaI
XbaI    Synthetic DNA(A)

EcoRI
BamHI
amp    trpA terminator
PvuII

T4 DNA ligase

EcoRI   trp P/O   XbaI
pT13S(Nco)
BamHI
amp    trpA terminator
PvuII

FIG. 12

Synthetic DNA(A)

```
                                                                    a                          b                                    21
                                                                                                                          Met  Ala
                                                           AACTAGTACGCAAGTTCACGTAAAAAGGGTATCGATAAGCC  ATG  GCA
                                                           TTGATCATGCGTTCAAGTGCATTTTTCCCATAGCTATTCGG  TAC  CGT
                                                                                              ClaI(TaqI)                   NcoI
                                                                                                                       1
```

```
                                         c                                                                              40        d
Pro  Thr  Ser  Ser  Ser  Thr  Lys  Lys  Thr  GlN  Leu  GlN  Leu  Glu  His  Leu  Leu  Leu  Asp  Leu  GlN  Met
CCT  ACC  TCG  AGT  AGT  ACT  AAG  AAA  ACA  CAG  CTG  CAG  CTA  GAG  CAT  CTG  CTG  CTA  GAT  CTC  GAG  ATG
GGA  TGG  AGC  TCA  TCA  TGA  TTC  TTT  TGT  GTC  GAC  GTC  GAT  CTC  GTA  GAC  GAC  GAT  CTA  GAG  GTC  TAC
          XhoI       ScaI(RsaI)            k    PvuII PstI   AluI                        BglII(Sau3A)                          j
          AvaI                                  (AluII)
```

```
                                              e
Ile  Leu  Asn  Gly  Ile  Asn  Asn  Tyr  Lys  AsN  Pro  Lys  Leu  Thr  Arg  Met  Leu  Thr  Phe  Lys  Phe  Tyr
ATT  TTG  AAG  GGA  ATT  AAT  AAT  TAC  AAG  AAT  CCC  AAG  CTT  ACG  CGT  ATG  TTA  ACA  TTT  AAA  TTT  TAC
TAA  AAC  TTA  CCT  TAA  TTA  TTA  ATG  TTC  TTA  GGG  TTC  GAA  TGC  GCA  TAC  AAT  TGT  AAA  TTT  AAA  ATG
                                           HinfI           HindIII              MluI        HpaI        DraI
                                                                           i                60
```

```
f
Met  Pro  Lys  Lys  Ala  Thr  Glu  Leu  Lys  His  Leu  GlN  Cys           78
ATG  CCT  AAG  AAG  GCC  ACA  GAG  CTG  AAG  CAT  CTT  CAG  TGT  GAT  C
TAC  GGA  TTC  TTC  CGG  TGT  CTC  GAG  TTC  GTA  GAA  GTC  ACA  CTA  G
          HaeIII        SacI                             XbaI
       h                                                            g
```

Synthetic DNA(A) = Artificial HIL-2 cDNA

Synthetic
   DNA(I)

5'                                                                          3'
CGA TAA GCC ATG ACC CTG CTG AAG AGG CTG CTC CAG CAG CA
   T ATT CGG TAC TGG GAC GAC TTC TCC GAC GAG GTC C
   3'                                                                   5'

FIG. 14  14/25

FIG. 15    0232707    15/25

Synthetic DNA (II)

```
5'                                                                           3'
  CGA TAA GCC ATG ATC TTT GGC TTC AGC GTG TCC CAT ACC ACG AGG AAC
    T ATT CGG TAC TAG AAA CCG AAG TCG CAC AGG GTA TGG TGC TCC TTG GGC T
  3'                                                                         5'
```

trp P/OClaI
EcoRI
SacI
HIL-2c DNA
pT13S(Nco)
amp
BamHI
trp A ter-
PvuⅡ   minator
PstI         Synthe-
tic DNA(Ⅲ)

trp P/O
EcoRI | Cla NcoI
HCGPFcDNA
pTHCGPF-01
amp
BamHI
PstI     PvuⅡ
EcoRI
BamHI

SacI
BamHI

amp
trpA terminator
PstI

HglAI

HglAI

T4 DNA ligase

trp P/O
pT13S₀HIL2(53)
-HCGPF-19    BamHI
amp         trp A terminator
PvuⅡ
PstI

Synthe-
tic DNA(Ⅲ)
          XbaI
    5'
      A  TCT AGA TTC CGC ACC CTG CTG AAG AGG CTG CTC CAG GAG CA  3'
   TC GAT AGA TCT AAG GCG TGG GAC GAC TTC TCC GAC GAG GTC C
 3'                                                          5'

          Phe Arg Thr Leu
                 19  20

                    HCGPh

kallikrein cleavage site

FIG. 17    17/25

0232707

without mouse IL3    with mouse IL3

X10³ cpm

Trithium thymidine uptake/5x10⁴ mouse bone marrow cells.

HCGPF sample (μg/ml)    HCGPF sample (μg/ml)

○ ΔHIL2(53)-HCGPF-19
⊙ ΔHIL2(20)-HCGPF-19
● ΔHIL2(11)-HCGPF-19
----- medium only

FIG. 18 18/25 0232707

Trithium thymidine uptake/5x10$^4$ mouse bone marrow cells

HCGPF samples (μg/ml)

o ΔHIL2(53)-HCGPF-19 6 M Guanidine HCl extract

⊙ same as above (CH$_3$CN=~5 5%)

--- mouse IL 3 only

Reverse HPLC fraction
(CH$_3$CN=~55 %)

FIG. 19

0232707

19/25

FIG. 20

DNA (Ⅳ)

```
5'                                                                              3'
   A TCT AGA TTC CGC ATC TTT GGC TTC AGC GTG TCC CAT ACC ACG AGG AAC
3' TC GAT AGA TCT AAG GCG TAG AAA CCG AAG TCG CAC AGG GTA TGG TGC TCC TTG GGC T 5'

            Phe Arg Ile Phe
                    31  32
                     |_____
                                          HCGPF

            kallikrein cleavage site
```

FIG. 21

FIG. 22    22/25

joining point

```
CTA GAT CCT AGA TTC CGC ACC CTG
GAT CTA GGA TCT AAG GCG TGG GAC
```

Leu·Asp Pro Arg Phe Arg Thr Leu
 19  20                  19   20

HIL-2                    HCGPF

kallikrein cleavage site

FIG. 23   23/25

joining point

```
CTA GAT C CT AGA TTC CGC ATC TTT
GAT CTA G GA TCT AAG GCG TAG AAA
Leu Asp pro Arg phe Arg Ile phe
 19    20              31   32

      HIL-2                    HCGPF
```

kallikrein cleavage site

FIG. 24    24/25

0232707

joining point

ACA CAG CCT AGA TTC CGC ACC CTG
TGT GTC GGA TCT AAG GCG TGG GAC

Thr Gln pro Arg phe Arg Thr Leu
10    11                    19    20

HIL-2                              HCGPF

kallikrein cleavage site

FIG. 25    25/25    0232707

$X10^4$cpm

Tritium thymidine uptake/5 x $10^4$ mouse bone marrow cells

3

without mouse IL3

2

1

0
X2  X4  X8  X16  X32  X64

folds dilution of
HCGPF samples

$X10^4$cpm

⊙: pKCR4-15 transfected
COS-7 supernatant

○: pKCR transfected COS-7
supernatant

●: medium only

with mouse IL 3

3

2

1

0
X2  X4  X8  X16  X32  X64

folds dilution of
HCGPF samples

**European Patent Office**

**EUROPEAN SEARCH REPORT**

. Application number

| | DOCUMENTS CONSIDERED TO BE RELEVANT | | EP 87100107.9 |
|---|---|---|---|
| Category | Citation of document with indication, where appropriate, of relevant passages | Relevant to claim | CLASSIFICATION OF THE APPLICATION (Int. Cl.4) |
| D,A | NATURE, vol. 309, June 28, 1984, New York, London<br><br>N.M.GOUGH et al. "Molecular cloning of cDNA encoding a murine haematopoietic, growth regulator, granulocyte-macrophage colony stimulating factor"<br>pages 763-767<br><br>* Totality *<br><br>-- | 1,14, 18,22, 26,43 | C 12 N 15/00<br>C 07 H 21/04<br>C 12 N 5/00<br>C 12 P 21/00<br>C 07 K 13/00<br>C 07 K 15/00 |
| A | WO - A1 - 85/04 188 (RESEARCH CORPORATION)<br><br>* Abstract *<br><br>-- | 1,14, 18,22, 26,43 | //(C 12 N 15/00<br>C 12 R 1:85)<br>(C 12 N 15/00<br>C 12 R 1:185) |
| D,A | SCIENCE, vol. 230, no. 4723, October 18, 1985, Washington DC<br><br>E.S.KAWASAKI et al. "Molecular cloning of a Complementary DNA Encoding Human Macrophage-Specific Colony-Stimulating Factor (CSF-1)"<br>pages 291-296<br><br>* Totality *<br><br>-- | 1,14, 18,22, 26,43 | |
| P,D, A | NATURE, vol. 319, no. 6052, January 30, 1986, New York, London<br><br>S.NAGATA et al. "Molecular cloning and expression of cDNA for human granulocyte colony-stimulating factor"<br>pages 415-418<br><br>* Totality *<br><br>-- | 1,14, 18,22, 26,43 | TECHNICAL FIELDS SEARCHED (Int. Cl.4)<br><br>C 12 N<br>C 07 H<br>C 12 P<br>C 07 K |

The present search report has been drawn up for all claims

| Place of search | Date of completion of the search | Examiner |
|---|---|---|
| VIENNA | 09-04-1987 | WOLF |

0232707

European Patent Office

**EUROPEAN SEARCH REPORT**

| DOCUMENTS CONSIDERED TO BE RELEVANT | | | EP 87100107.9 |

| Category | Citation of document with indication, where appropriate, of relevant passages | Relevant to claim | CLASSIFICATION OF THE APPLICATION (Int. Cl.4) |
|---|---|---|---|
| D,A | NATURE, vol. 307, no. 5948, January 19, 1984, New York, London M.C.FUNG et al. "Molecular cloning of cDNA for murine interleukin-3" pages 233-237 * Totality * | 1,14, 18,22, 26,43 | |
| A | US - A - 4 438 032 (GOLDE et al.) * Claims * | 1,14, 18,22, 26,43 | |

TECHNICAL FIELDS SEARCHED (Int. Cl.4)

The present search report has been drawn up for all claims

| Place of search | Date of completion of the search | Examiner |
|---|---|---|
| VIENNA | 09-04-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82